# EUROPEAN PATENT APPLICATION

(11) **EP 3 175 881 A1**
(43) Date of publication of application: **07.06.2017**
(21) Application number: 16201743.8
(22) Date of filing: 01.12.2016
(51) Int. Cl.: A61M 25/06

(54) **NEEDLE DEVICES WITH BISTABLE NEDDLE GUARD STRUCTURE AND RELATED METHODS**

(30) Priority: 01.12.2015 US 201562261770 P
(71) Applicant: B. Braun Melsungen AG, 34212 Melsungen (DE)
(72) Inventor: SONKSEN, Julian, 34212 Melsungen (DE)
(74) Representative: Kinkeldey, Daniela

(57) **Abstract**

A needle device having a needle guard (122) located in a first hub (152) mounted over a needle (104) having a second hub (154), a shaft, a needle tip (110) and a change in profile (128) located proximal of the needle tip (110). The needle guard (122) has a proximal wall (126) having a perimeter defining an opening therein, a first arm (20) extending distally from the proximal wall (126), and a second arm (30) extending distally from the proximal wall (126) opposite the first arm (20). The first arm (20) and the second arm (30) are spaced from the needle (104) in a ready to use position. The first arm (20), the second arm (30), or both arms cover the needle tip (110) in a protective position when the needle guard (122) is triggered by an activator (140).

## Description

### FIELD OF ART

The disclosed invention generally relates to needle safety devices and more specifically to needle guards that cover needle tips following activation to prevent inadvertent needle sticks.

### BACKGROUND

Insertion procedure for an IV catheter assembly contains four basic steps: (1) the healthcare worker inserts the needle and catheter together into the patient's vein; (2) after insertion into the vein with the needle point, the catheter is forwarded into the vein of the patient by the healthcare worker pushing the catheter with his or her finger; (3) the healthcare worker withdraws the needle by grasping the hub end (opposite the point end) while at the same time applying pressure to the patient's skin at the insertion site with his or her free hand to stop the flow of blood through the catheter; and (4) the healthcare worker then tapes the exposed end of the catheter (the catheter hub) to the patient's skin and connects it to the source of the fluid to be administered into the patient's vein.

The problem is that immediately after the withdrawal of the needle from the patient's vein, the healthcare worker, who is at this time involved in at least two urgent procedures, must place the exposed needle tip at a nearby location and address the tasks required to accomplish the needle withdrawal. It is at this juncture that the exposed needle tip creates a danger of an accidental needle stick, which, under the circumstances, leaves the healthcare worker vulnerable to the transmission of various dangerous blood-borne pathogens, including AIDS and hepatitis.

Other needle types similarly expose healthcare workers to risks of accidental needle sticks. For example, a doctor administering an injection, using a straight needle, a Huber needle, an epidural needle, etc., may place the used needle on a tray for subsequent disposal by a nurse. During the period between placing the used needle on a tray or a work station to the time it is discarded, the used needle is a potential source for disease transmissions for those that work near or around the needle.

Accordingly, all needles should be covered immediately following use to ensure greater worker safety. Ideally, the procedure for covering the needle tip should be passive, self-activating, or at least simple to perform. In addition, the device for covering the needle should be reliable and robust.

Needle devices often include safety systems that cover the tip of the needle to prevent accidental sticks after placement of the catheter tube into the vasculature of a patient. These systems can be either passive or active. In some systems, the safety features are located inside the catheter hub in the ready position while in other systems they are external of the catheter hub. In either location, the safety features serve the same function, to cover the needle tip in order to prevent accidental needle sticks after venipuncture. Thus, great care must be exercised when installing the safety systems to prevent dimension changes or deformation which can hamper or hinder the function and performance of the safety systems.

### SUMMARY

Aspects of the present disclosure include needle devices and related methods of forming and using the needle devices.

An exemplary needle device can be a needle assembly or an IV catheter assembly. The needle device can also be an over-the-needle catheter assembly.

The needle device can have configurations as described in the various embodiments or can be varied by incorporating features from the described embodiments.

In an example, the needle device can comprise a first hub having a hub body with a proximal end, an interior cavity, a catheter tube extending distally from a distal end of the catheter hub, a second hub attached to the proximal end and having a cannula or needle extending distally of the needle hub and projecting through the proximal end and the catheter tube and having a needle tip of the needle extending distally of a distal opening of the catheter tube. The needle device can be in a ready to use position when the needle tip extends distally of a distal end of the catheter tube for accessing the vasculature of a patient.

The first hub can be a catheter hub and the second hub can be a needle hub.

A needle guard or spring clip can be slidably positioned on the needle and located in the interior cavity of the catheter hub in the ready to use position. In other examples, a third hub or a guard housing can be positioned between the first hub and the second hub. The needle guard can be positioned in the third hub.

A trigger or activator can be located in the interior cavity distal of the Luer taper of the catheter hub. The trigger can activate the needle guard. The trigger can instead locate outside of the catheter hub.

A proximal end of the catheter tube can attach to the catheter hub using a bushing or other conventional means.

In the ready position, the needle can project distally from a nose section of the needle hub through the catheter hub. The catheter hub can have wings, can be a ported catheter hub, or can be provided with an integrated extension system, such as via a side port.

The needle can have a needle tip extending out a distal end of the catheter tube.

A proximal end of the needle can extend into a flashback chamber of the needle hub, which can have an opening at a proximal end that can be closed by a vent plug.

Optionally, the vent plug can have sampling features that allow blood to be collected and dispensed.

The blood can be collected and dispensed on a glucose strip or a Petri dish.

An exemplary blood stopper can be similar to the blood stopper disclosed in pending U.S.

Patent Application No. 14/576,802, filed December 19, 2014, published as Publication No. US2015/0173663.

The needle tip can be configured for penetrating the epidermal layer of a patient and accessing the vasculature of the patient.

The needle hub can have a needle guard extension extending from the nose section into the open chamber of the interior cavity of the catheter hub for aligning the needle guard during assembly.

The proximal end of the catheter hub can have a female Luer taper with external threads. In one example, the proximal end of the catheter hub can be a threaded female Luer connector.

The female Luer connector can be configured to matingly receive a male Luer connector, such as an IV line, a Luer access connector, a syringe tip, a vent plug, an IV set, an extension set, another known connector, or future-developed IV devices with a Luer tip. Each of these components can be sized and configured in conformity with at least some of the International Standards Organization (ISO) standards for female and male Luer connections under current or future standards.

The threaded female Luer connector can receive a nose section of the needle hub or other male Luer tips.

A physical abutment can be provided between the catheter hub and the needle hub so as to set the distance the needle tip extends out the distal end of the catheter tube.

The catheter hub can include a tab on an exterior thereof configured for use as leverage when handling the device, such as to push against during insertion or removal of the needle.

A pair of wings may extend laterally of the catheter hub to support and stabilize the catheter hub against the patient following successful venipuncture.

The tab and the wings are optional features. Either the tab or the wings, or both the tab and the wings can be omitted.

A trigger or activator can be incorporated for triggering the needle guard to change its shape from an extended structural configuration to a coiled structural configuration to cover the needle tip in a protective position when the needle guard is withdrawn proximally from the ready to use position after successful venipuncture.

The trigger can be unitarily formed with the catheter hub or separately formed and subsequently attached to the catheter hub. The trigger can alternatively be located external to the catheter hub.

In an example, when the needle guard is in an extended structural configuration, the distal ends of the two arms of the needle guard can be spaced from the needle shaft so that no drag is exerted on the shaft by the flaps or arms of the needle guard when the needle moves relative to the needle guard, such as following placement of the catheter tube in a patient's vasculature.

In other examples, no part of the two arms of the needle guard can contact the shaft in the extended structural configuration. In an example, the extended structural configuration can be a ready to use position of the needle. The extended structural configuration can be the transition position wherein the needle retracts from the catheter hub following successful venipuncture.

The activator can be located in the interior cavity of a catheter hub by pressing a generally cylindrical outer surface of the activator against the interior wall surfaces of the interior cavity, and distal of the female Luer chamber of the catheter hub.

The shape or contour of the outer surface of the activator can match the surface of the interior cavity, such as being straight or taper.

In one example, the outer surface can include surface features to align the activator in a particular orientation inside the interior cavity.

In one example, a notch and a groove can be incorporated between the activator and the interior cavity to facilitate orienting the activator inside the catheter hub.

A projection, which can act as a stop, can be included inside the interior cavity to prevent the activator from displacing proximally out of position from a set position inside the interior cavity. The activator can alternatively be bonded or co-molded to the hub body.

The projection, if incorporated, can embody a continuous protrusion formed around a periphery or interior diameter of the interior cavity.

In another example, the projection can comprise a single bump or two or more spaced apart sections formed around a periphery of the interior cavity instead of a continuous annular ring.

The projection can have two surfaces forming an apex wherein the two surfaces can include a blocking surface and an inclined surface proximal of the blocking surface. The projection can define a minimum interior dimension.

The inclined surface can elastically deform the activator as the activator is pushed distally inside the interior cavity over the inclined surface and past the apex.

Once the activator has cleared the apex, the activator can snap back into its original shape with the proximal end of the activator contacting the blocking surface.

The blocking surface can prevent the activator from moving proximally past the blocking surface when the needle guard is retracted proximally during retraction of the needle following successful venipuncture.

In one example, the activator can be press fitted inside the interior cavity thus eliminating the need for the projection.

In other examples, the activator can be pressed fit inside the catheter hub and a projection can also be incorporated.

The activator can be made from a number of materials, including from plastic or metal.

The activator can have a distal face at a distal end, a proximal face at a proximal end, and a bore extending along a lengthwise axis of the activator through the distal face and the proximal face.

The distal face can be flat or have a conical shape which tapers inwardly or outwardly. The proximal face can also be flat or have a conical shape which tapers inwardly so as not to interfere with a male Luer taper inserted into the Luer chamber through the proximal opening.

The shapes of the distal face and the proximal face are not limited and can take on a variety of shapes to serve any purpose.

In other examples, the activator can be hollow and/or made from a resilient material that readily deforms when pushed into the bore of the catheter hub, such as from a polymer material.

The bore of the activator can be sized and shaped to activate or trigger the needle guard from the extended configuration to the coiled configuration and to allow the needle guard and the needle, including the needle tip, to pass through the bore of the activator in the proximal direction when the needle guard is in the protective position blocking the needle tip.

The bore of the activator can be a straight bore or a tapered bore. The bore can taper inwardly in the proximal direction so that the opening at the proximal end of the bore is smaller than the opening at the distal end of the bore.

When the needle guard of the present disclosure changes from an extended configuration to a coiled configuration or when the needle guard changes from the coiled configuration back to the extended configuration, the needle guard can undergo a change in state or a phase change.

In one example, the needle guard in the extended configuration cannot pass through the bore of the activator without contacting the activator.

The bore of the activator can be rectangular in shape and can have a length that tapers inwardly from the distal face in the proximal direction.

The interior surfaces of the bore can be flat, concave, or convex. In other examples, the bore of the activator can be generally cylindrical with a taper lip at the entrance.

The bore of the activator can be sized and shaped to activate the needle guard and to allow the needle guard to pass from a distal position of the activator to a proximal position of the activator, such as following successful venipuncture and as the needle is retracted away from the catheter hub with the needle guard covering the needle tip.

Activating edges formed between a surface of the bore of the activator and the proximal face of the activator can be configured to engage and trigger the needle guard to change its configuration as the needle guard is withdrawn proximally.

The activating edges of the activator can be flat, concave, or convex. The activating edges can be located along the same axial position of the activator or along different axial positions of the activator to contact and activate the needle guard at a different time and/or a different location of the needle guard.

Other surfaces and features of the activator or bore of the activator can be configured to change the configuration of the needle guard.

In one example, the needle guard can contact a surface of the bore of the activator other than the proximal activating edges of the activator to change the configuration of the needle guard from the extended structural configuration to the coiled structural configuration.

The needle guard can be positioned completely inside the interior cavity of the catheter hub in the ready position and during needle withdrawal prior to securing the needle tip with the needle guard in the protective position.

In other examples, the needle guard can be positioned partially inside the catheter hub body or completely outside the catheter hub, such as in a shroud or a separate needle guard housing between the catheter hub and the needle hub, so long as the needle guard can be activated to a coiled position by the activator to prevent exposure of the needle tip.

In an example, the needle guard can comprise a proximal wall and two flaps such as a first or top flap and a second or bottom flap, extending distally of the proximal wall.

The flaps of the needle guard can have the same width or different widths and the same length or different lengths.

The flaps of the needle guard may alternatively be labeled or called arms.

A distal extension or extension can extend from a distal end of the top flap of the needle guard or on an end opposite the proximal wall of the needle guard.

The extension of the needle guard can also extend from the side flaps of the needle guard.

The distal extension of the needle guard can include one or more bent sections, which can extend towards the needle or remain parallel with the top flap of the needle guard.

In another example, a second distal extension can extend from a distal end of each of the two flaps of the needle guard or from the side flaps of both the upper and lower arms of the needle guard.

The flaps of the needle guard can be spaced from the interior wall surfaces of the interior cavity, the needle, and the activator in the ready to use position.

During needle retraction, the needle guard can slide proximally and contact the activator but not actuated by the activator until after being pulled in the proximal direction by the change in profile on the needle against a proximal wall of the needle guard and moving the needle guard into the activator.

In other examples, during retraction, the needle guard can be spaced from the activator and not touch the activator so that the needle can move within the opening on the proximal wall until the change in profile on the needle engages the proximal wall then moving the proximal wall and hence the needle guard into the activator.

The length of the needle guard can be greater than a distance between the change in profile and the needle tip to ensure capturing of the needle tip in the needle guard in the protective position.

The proximal wall of the needle guard can comprise a perimeter defining a proximal opening having the needle passing therethrough.

The perimeter of the proximal opening in the proximal wall of the needle guard can be configured for engaging the change in profile of the needle following placement of the catheter tube into a patient's vasculature.

The change in profile of the needle can be a crimp, a bulge, a sleeve, or a material buildup formed on the shaft of the needle adjacent the needle tip to prevent the needle guard from displacing distally off of the needle in the protective position.

In one example, the length of one of the flaps of the needle guard can be greater than the length of the other of the flaps of the needle guard.

The widths of the two flaps of the needle guard can be the same or different.

Having different lengths can allow the two flaps of the needle guard to stagger when in the coiled configuration.

In another example, the length of the two flaps of the needle guard can be substantially the same.

Each of the top flap and the bottom flap of the needle guard can have one or more sidewalls extending from opposite sides of the top flap and the bottom flap.

The one or more sidewalls of the needle guard can extend towards each other.

The length, width, and shape of the sidewalls of the needle guard can be uniform or can vary.

The two flaps, the proximal wall, and the sidewalls of the needle guard can define a needle tip holding space when the needle tip is located therein, such as when the needle guard is activated and covers the needle tip.

The sidewalls of the needle guard can prevent the needle from moving sideways out of the sides of the needle guard and out of the needle holding space.

The sidewalls of the needle guard can increase the structural rigidity of the flaps of the needle guard.

Along a side of each flap of the needle guard, a gap can be provided between two adjacent sidewalls of the needle guard.

The gap of the needle guard can be trapezoidal, triangular, rectangular, or other shapes that provide sufficient space or room for the sidewalls to move when the flaps of the needle guard are activated.

The dimension and shape of each gap of the needle guard can vary depending on the shape of the adjacent sidewalls of the needle guard and the extent or amount of movement of the flaps of the needle guard following activation.

At least one of the top flap and the bottom flap of the needle guard can have an arcuate surface along their respective widths.

If each flap of the needle guard has a length along the lengthwise axis of the needle and a width orthogonal thereto, the side of the needle guard can have an arcuate surface.

In some examples, the entire width of each flap of the needle guard can be formed along an arc or a curve.

In some examples, the entire width of one or both flaps of the needle guard can be flat or not curved.

Both the top flap and the bottom flap of the needle guard can have an arcuate surface along the width of the top and bottom flaps. The arcuate surface of the top flap and the bottom flap can be concave or convex and can extend the entire length of the flap distal of the proximal wall.

The wall surface of the flaps of the needle guard can have a substantially constant thickness and the arcuate surface can have a substantially constant radius of curvature or can have a complex curve.

In other examples, the flaps of the needle guard can have a variable thickness and the radius of curvature of the arcuate surface can vary along the width.

The arcuate surface of the flaps of the needle guard can force the flaps to extend distally in the extended structural configuration along a lengthwise direction.

By maintaining an arc along the width for the length of the flap of the needle guard, the arcuate surface can make the flaps stable in the extended configuration.

The arc-shape structure can provide reinforcement and stiffness that allow the flap of the needle guard to extend axially without breaking, collapsing, distorting, buckling or otherwise undergo a phase change until activated by a sufficient force, such as by an activator.

The sidewalls along the lengthwise direction of the flaps of the needle guard can stiffen the flaps by increasing the moment of inertia. Said differently, the presence of the sidewalls can prevent portions of the flaps of the needle guard from transitioning into the coiled configuration.

The structural rigidity of the flaps of the needle guard along the activating portions can be altered between the extended configuration and the coiled configuration by providing gaps in the sidewalls along the lengthwise direction of each flap.

The gaps can be used to control or select the activating portions of the flaps of the needle guard to transition to a coiled configuration when triggered by the activator.

In some examples, sections of the flaps of the needle guard may not roll so that sections of the flaps, even without the side walls, can remain straight and not coil when activated.

The location and shape of the sidewalls and gaps on the flaps of the needle guard can control the shape of the needle guard in the coiled configuration and therefore how the flaps in the coiled configuration can cover the needle tip in the protective position.

The structure that can morph between one stable structural configuration or state to another stable structural configuration or state can be a bistable structure or a structure with bistable phases.

The needle guard can be formed from a body having an arcuate surface along a width or along a width section, which is generally orthogonal to the length.

The body of the needle guard can be stamped or cut in the unfolded state from a length of metal sheet or other material having an arcuate surface along the width.

The body of the needle guard and the various cut-out sections of the body can be folded or manipulated to form various sections of the needle guard.

In one example, the body of the needle guard can be generally rectangular having a length L, a width W extending orthogonally of the length, a substantially constant thickness T, and an arcuate surface along the width.

The arcuate surface can have a substantially constant radius of curvature R along the width.

The arcuate surface can have a complex curve and the shape can extend along the lengthwise direction of the body.

The arcuate shaped width of the body of the needle guard can have a concave surface and a convex surface.

Depending on the direction the body of the needle guard is coiled in the lengthwise direction to create the second bistable state, the concave surface can be located to the outside or the convex surface can be located to the outside when the body is activated into its coiled configuration.

The shape of the body of the needle guard can be generally rectangular.

In other examples, the shape of the body of the needle guard can have a regular or irregular polygonal shape

In yet other examples, the shape of the body of the needle guard can comprise multiple pieces attached or pieced together.

The thickness of the body of the needle guard can be constant.

In another example, the thickness of the body of the needle guard can vary, and the radius of curvature can be constant along the widthwise direction.

In other examples, the radius of curvature can vary along the widthwise direction, such as having a complex curve.

One or more sections of the body of the needle guard can be a bistable structure, which means that the one or more sections of the body can morph between two stable structural configurations.

In one example, a width section of a flap or both flaps of the needle guard can alternate from a first shape to a second shape and wherein the first shape can be characterized by a curved surface and the second shape can be characterized by a surface that is less curved, such as being more flat or generally flat.

An example of a bistable structure can be a slap bracelet, which is widely commercially available as kids' toys and reflectors for cyclists.

The two stable configurations of the needle guard can be stabilized by different curvature axes. A first stable configuration of the two stable mechanical configurations can be the extended configuration and the second stable configuration can be the coiled configuration.

In the extended configuration, the arcuate surface of the body of the needle guard can force the body in a straight extended configuration along the lengthwise direction. That is, the arcuate surface of the body can force the body to be stable in the extended configuration.

The body of the needle guard can remain in the extended configuration until an external force is applied to the body, which can then transition the body from the first stable configuration (extended configuration) to the second stable configuration (coiled configuration).

In one example, a force, such as an impulse force, can be applied to flatten or bend any part of the concave cross-section of the body of the needle guard to change the body into its coiled configuration.

In the extended configuration, the width of the flap of the needle guard can have a bend or arc section, and when activated to the coil configuration, the width can flatten out, or at least can have less of a curve, less of a bend, or less of an arc shape than when in the extended configuration, as the length of the body coils up to the second stable configuration.

Once in transition, the body of the needle guard can continue transitioning from the extended configuration to the coiled configuration without additional force because internal stresses in the body can cause the body to morph from the extended configuration into the coiled configuration.

During the transition, the arcuate surface of the body of the needle guard can flatten thereby forcing the body to curve or coil along an axis different than the axis of the arcuate surface. In one example, the coiling axis can be perpendicular to the arcuate surface axis.

A further aspect of the present disclosure can include a needle device comprising a catheter hub having a body defining an interior cavity and having a proximal opening, a catheter tube extending distally of the catheter hub and comprising a distal opening, a needle hub, a needle extending distally of the needle hub and through said catheter hub and said catheter tube in a ready to use position.

The needle can comprise a shaft, a needle tip extending distally of the distal opening of the catheter tube, and a change in profile proximal of the needle tip.

The needle can pass through a needle guard comprising a proximal wall having a perimeter defining an opening, a first arm extending distally of the proximal wall, and a second arm extending distally of the proximal wall opposite the first arm.

The first arm and the second arm of the needle guard can be spaced from the needle in the ready to use position and the needle guard in a first configuration, and can cover the needle tip in a protective position when the first arm and the second arm are activated.

A part of a width of the first arm, the second arm, or both arms of the needle guard can change from a first shape to a second shape having less of a curved surface when the needle guard is in a second configuration.

In some examples, the needle guard can be used with a hub with an interior cavity that is not a catheter hub.

In the coiled configuration, the body of the needle guard can be coiled along the lengthwise direction.

The coiled body of the needle guard can be uniform or non-uniform in which certain sections coil more than others or where some sections are not coiled at all.

In a coil stated, the body of the needle guard may not be circular or round but can have a polygonal shape or an irregular rolled up shaped. The body can also be stable in this coiled configuration.

The body of the needle guard cannot transition back to the first stable configuration unless an external force is applied to uncoil the body back into the extended configuration. The body can transition between these two stable configurations, phases, or states.

Once the body of the needle guard returns to the extended configuration from the coiled configuration, the body can remain stable in the extended configuration until a force is again applied to the body, which can then transition the body back to the coiled configuration.

In one example, the coiled configuration may be coiled in a coiling direction against or away from the arcuate surface.

The body of the needle guard can be coiled such that when activated from the extended configuration to the coiled configuration, the concave surface can be located to the exterior or outside of the coiled shaped structure.

In another example, the coiled configuration of the needle guard may be coiled in the opposite direction such that the convex surface is now located to the exterior of the coiled shaped structure when the body is activated from the extended configuration to the coiled configuration.

The arcuate surface of the body of the needle guard, such as the width of the body, can flatten to maintain a substantially flat cross-section in the coiled configuration with either the concave surface located on the exterior or the convex surface on the exterior.

The substantially flat cross-section combined with the coiling of the body of the needle guard along an axis different to the axis of the arcuate surface can allow the body to be stable in the coiled configuration.

In some examples, the shape can be less curved or can have less of a bend without completely or substantially flattened out.

The flaps or other sections of the needle guard can have bistable configurations that allow the needle guard, such as one or both flaps or sidewalls, to be arranged in a ready to use position inside a catheter hub and a folded or coiled configuration to cover the needle tip following successful venipuncture.

The needle guard with bistable configuration may be used with any number of needle devices, with or without a catheter tube, such as fistula needles.

The various sections of the body can be manipulated, such as bent or curved, to include the proximal wall, the bottom flap extending distally of a first connecting side of the proximal wall, the top flap extending distally of a second connecting side of the proximal wall opposite the first side, the optional distal extension extending from the top flap, and one or more sidewalls extending from opposite sides of the flaps with gaps provided adjacent to or between one or more sidewalls.

The portions of the flaps of the needle guard coinciding with the gaps can be defined as activating portions. The width or spacing of each gap and the number of gaps can be selected depending on the shape of the coiled configuration of one or more flaps, as desired.

In other examples, the distal extension of the needle guard can extend from other sections of the needle guard, such as from the bottom flap or from the one or more sidewalls.

In some examples, sidewalls of the needle guard can be incorporated to prevent the needle tip from protruding out a side of the flaps of the needle guard and stiffen the flaps at portions of the flaps bordered by the sidewalls.

The portions of the flaps of the needle guard bordered by the sidewalls can be defined as non-activating portions.

The portion of the body of the needle guard representing the proximal wall can be generally rectangular with the connecting sides each extending along a widthwise direction and having an arcuate cross-section.

Because the connecting sides of the needle guard can have arcuate cross-sections, cutouts can be provided extending inward from opposite sides of the connecting sides to ease bending along the connecting sides. In one example, the cutouts can be formed along upper and lower edges of the proximal wall of the needle guard, near the two connecting sides. In another example, four cutouts can be located between the proximal wall and the two flaps.

Folding lines can be provided on the body of the needle guard to designate foldable or desired locations when forming the needle guard.

In one example, the folding lines can be etched or marked on the body of the needle guard.

The needle guard can be formed by folding the body along the folding lines.

In one example, the body of the needle guard can be folded with the arcuate surfaces of the top flap and the bottom flap of the needle guard opening outwardly in a direction away from the needle.

In another example, the body of the needle guard can be folded with the arcuate surfaces of the top flap and the bottom flap of the needle guard opening in a direction towards the needle.

The arcuate surfaces of the flaps of the needle guard are concave with the curve facing outwardly in a direction away from the needle.

In one example, only the flaps of the needle guard can have arcuate surfaces, and not the proximal wall.

The separately formed flaps of the needle guard can be separately attached, directly or indirectly, to the proximal wall, such as by welding.

The one or more sidewalls of the needle guard can be bent towards each other along the folding lines.

The sidewalls and the gaps along the flaps of the needle guard can be arranged and shaped to ensure that when activating portions of the flaps, the sidewalls do not interfere with each other and obstruct the flaps' ability to coil.

The sidewalls of the needle guard may also be bent outwardly or bent multiple times.

The distal extension of the needle guard can be bent towards the bottom flap or remain parallel with the top flap.

The distal extension of the needle guard can be incorporated for securing or locking the top flap to the bottom flap and/or to obstruct the needle tip from re-emerging out the distal end of the needle guard following activation thereof.

After the needle guard is formed, the needle guard can be assembled onto the needle device along with the activator and prior to assembling the needle to the needle hub.

In other examples, the needle guard can be mounted onto the needle after the needle is attached to the needle hub.

In one example, the crimp or change in profile on the needle can be formed after the needle guard is slidably placed onto the needle.

The needle can be retracted proximally away from the catheter hub following successful venipuncture by pulling on the needle hub in the proximal direction.

Because the opening on the proximal wall is larger than the diameter of the shaft of the needle, the needle can be retracted proximally without dragging or pulling on the needle guard proximally from a needle guard set position or from the catheter hub.

As the flaps of the needle guard are spaced from the needle in the ready to use position, the needle can also be retracted following successful venipuncture without the needle shaft or the change in profile contacting the flaps.

The needle guard or the flaps can remain in the extended configuration while the needle retracts in the proximal direction until the change in profile engages the perimeter of the opening in the proximal wall.

With the change in profile on the needle engaged against the perimeter defining the opening of the proximal wall of the needle guard, further retraction of the needle can pull the needle guard proximally towards activating the activator.

When the flaps of the needle guard engage the activating edges or other surface features of the activator and further movement of the needle guard in the proximal direction against the resistance of the activator continues, the contact forces experienced by the flaps can cause the flaps to move inward towards the needle. The contact forces can also cause the bistable nature of the flaps to undergo a change.

The flaps of the needle guard can transition from the extended configuration to the coiled configuration to cover the needle tip when activated.

When the needle guard is triggered, the activating portions of the flaps can coil inwardly to cover the needle tip in the protective position.

The activating portions of the flaps of the needle guard can transition into a relatively flat surface along their respective widths and coil towards the needle to cover the needle tip.

The portions of the flaps of the needle guard that do not undergo a change in state can remain in the extended configuration.

In one example, because of the increased rigidity from the sidewalls, and because of no rolling to create bi-stable phases or both, portions of the flaps that border the sidewalls may not experience a phase change.

The sidewalls can prevent the portions of the flaps of the needle guard bordering the sidewalls from coiling.

In one example, the sidewalls and the gaps along the flaps of the needle guard can be positioned and shaped to ensure that when portions of the flaps coil or undergo a phase change, the sidewalls do not interfere with each other or interfere with the portions of the flaps that are intended to undergo a change.

In other examples, the sidewalls and the gaps of the needle guard can be designed to engage each other to interlock the flaps of the needle guard together when the needle guard is in the coiled configuration to cover the needle tip in the protective position.

The distal ends of the flaps of the needle guard can cooperatively cover the needle tip in the protective position to prevent the needle tip from re-emerging distally out the needle guard.

If the top flap of the needle guard is longer than the bottom flap of the needle guard, the bottom flap can transition to the coiled configuration before the top flap to ensure the top flap covers the bottom flap in the protective position.

In other examples, the bottom flap of the needle guard is longer than the top flap of the needle guard and the top flap can transition to the coiled configuration before the bottom flap to ensure the bottom flap covers the top flap in the protective position.

The sequence of how the flaps of the needle guard transition or undergo a phase change can be controlled by selecting where to place the sidewalls, where the flaps contact the activator, the location and shape of the activator, the number of gaps, the size of the sidewalls, or combinations thereof.

The distal extension at the distal end of the top flap of the needle guard can engage with the bottom flap of the needle guard to help secure the needle tip.

In one example, the distal extension of the needle guard can wrap below the bottom flap of the needle guard to lock the needle guard and secure the top flap and the bottom flap together.

In another example, an additional distal extension can extend distally from the bottom flap to create additional friction between the two flaps to help secure the top flap and bottom flap together.

Once the needle guard covers the needle tip in the protective position, the needle guard can be pulled away from the activator allowing the needle to separate from the catheter hub with the needle guard covering the needle tip.

A fistula needle assembly, which can be used in dialysis access, can comprise a first hub and a second hub removably engaged to one another in a ready to use position, which is a position in which the needle assembly is ready for use on a patient.

The second hub can have a needle with a change in profile proximal of a needle tip extending therefrom.

The first hub can comprise a body defining an interior cavity having a tip protector or needle guard located therein.

The second hub, which may also be referred to as a needle hub, can have a body defining an interior cavity having a proximal opening having a vent plug attached thereto.

The interior cavity may be referred as a blood flashback chamber.

The vent plug can include a vent filter at a proximal end thereof for venting air or gas but not fluid, such as blood.

A nose section of the second hub can extend into a proximal opening of the first hub to removably secure the two hubs together in the ready to use position.

In other examples, the two hubs can removably engage one another in the ready to use position using other means, such as by gripping the external surface of the first hub with a shroud on the second hub.

A pair of wings can extend laterally of the body to provide points for securing the second hub to the patient.

The tip protector can have bistable configurations, phases, or states.

The tip protector can be used with a needle without an over-the-needle tube, such as a catheter tube.

The tip protector can be located in the interior cavity of the first hub, which has a proximal opening and a distal opening. The interior cavity can define a bore that is accessible through the proximal opening and/or the distal opening.

The distal end of the first hub can have a distal wall to partly close the distal opening such that a smaller hole is provided as the distal opening that is sufficiently large for the needle to pass therethrough but not the needle guard.

The proximal opening of the first hub can be sufficiently large to allow the needle guard to pass proximally thereof in a protective position to separate from the first hub after activation and removed from the proximal opening of the catheter hub.

Interiorly, the first hub can have a trigger or activator for activating the needle guard, such as to change the state of the needle guard from an extended state or first configuration to a coiled state or second configuration.

The trigger or activator may be retained in the bore by a projection.

The first housing and the activator can be unitarily formed.

The first housing and the activator can be unitarily formed by plastic injection.

In other examples, the activator can be separately formed and subsequently assembled to the first hub.

In use, the needle can be inserted to gain access to the patient's venous system. After use, the needle can be retracted away from the patient by pulling on the second hub in the proximal direction while the first hub is held so that the needle moves relative to the first hub and the needle guard.

The needle can move until the change in profile abuts a perimeter defining an opening on the proximal wall of the needle guard, which is smaller in dimension that the largest width at the change in profile so that a physical contact is made by the change in profile at the opening.

The needle can the pull the needle guard in the proximal direction against the activator, which can cause the needle guard to change its state from a first or extended state to a second or coiled state to cover the needle tip.

The needle guard can separate from the first hub with the needle.

The first hub and the protected needle having the needle guard covering the needle tip can be disposed of via standard protocol.

The needle can be removed from the patient before activating the needle guard by holding the needle hub or the second hub steady while advancing the first hub with the needle guard located therein distally relative to the needle towards the needle tip so that the proximal wall abuts the change in profile. Further movement at this point can force the activator inside the first hub to contact and cause the needle guard to change its state from a first or extended state to a second or coiled state to cover the needle tip.

The second hub can be retracted in the proximal direction while the first hub is moved in the distal direction to activate the needle guard.

A venous needle assembly and an arterial needle assembly can be arranged in a conventional manner on an arm with a dialysis shunt for hemodialysis.

The venous needle assembly can be the same as the fistula needle assembly.

The arterial needle assembly can be a conventional needle assembly for use in hemodialysis.

A tether can be connected to the first hub to secure the first hub while a user or practitioner retracts the needle with a needle tip in the proximal direction relative to the first hub following dialysis.

The user or practitioner can retract the needle by gripping and moving the second hub away from the first hub to allow the change in profile on the needle to be moved proximally against the proximal wall of the needle guard and then pulling or moving the needle guard proximally against the activator to activate the needle guard.

The needle guard can change state from a first or extended state to a second or coiled state to cover the needle tip by using a tether to hold the first hub steady and permit relative movement between the first hub having the needle guard located therein and the needle.

The tether may have a first end connected to the first hub and a second end connected to an arterial needle assembly.

The tether may be made from any conventional prior art material, including from a fiber material, a plastic material, or a thin gauge wire.

The ends of the tether can be secured by adhesive, welding, knotting, or combinations thereof.

One of the ends of the tether can be connected to an anchor instead of the arterial needle assembly.

In an example, the anchor can be a strap, an adhesive strip, or other securement means for holding the end of the tether steady against the force of the retracting needle.

As the needle is retracted, the anchor holds the tether thus preventing the first hub from moving so that the needle can move relative to the first hub to move the change in profile on the needle against the proximal wall of the needle guard.

The needle guard can change state from a first or extended state to a second or coiled state to cover the needle tip by using a tether and an anchor to hold the first hub steady and permit relative movement between the first hub having the needle guard therein and the needle.

The first hub can be secured by an adhesive structure, such as a tape, a two-sided tape, an adhesive pad, or combinations thereof.

As the needle is retracted, the adhesive structure can hold the first hub from moving so that the needle can move relative to the first hub to move the change in profile on the needle against the proximal wall of the needle guard.

The needle guard can change state from a first or extended state to a second or coiled state to cover the needle tip by using an adhesive structure to hold the first hub steady and permit relative movement between the first hub having the needle guard therein and the needle.

In other examples, a strap or a band structure can be secured to a patient's arm and the strap or a band structure can have a receiving bay for receiving the first hub to secure the first hub and allowing relative movement between the first hub and the needle.

The strap or band with the bay can wrap around the arm or can be secured to the arm with adhesive.

A needle guard having bistable configurations, phases, or states can be located inside a hub having an activator and mounted on a needle.

The needle guard can be activated to change state from a first or extended state to a second or coiled state.

The first and second arms of the needle guard can be sized and shaped to coil into a gate to close or block the bevel at the needle tip when the needle guard is activated by an activator.

In an example, a gap can be created between each of two sets of sidewalls on the first arm.

Each of the gaps can be axially located on the first arm so that when the first arm is activated, a section of the first arm having a length can be slanted to form the gate for blocking the needle bevel.

The length can be selected by selecting where to form the gaps so that a bend at or near the apex of the gap extending to a tip near the extension can be sufficiently long so as to extend across the entire bevel of the needle.

The sidewalls on the second arm can be provided with a distal mating edge.

When the gate rotates to cover the bevel, the gate can abut or contact the two distal mating edges of the second arm.

The second arm can be provided with a sidewall extending from each of the two side edges of the second arm and each with a distal mating edge.

The sidewalls can be provided without any gap so as to form rigid sections to prevent the second arm from activating.

In other examples, the sidewalls of the second arm can include one or more gaps formed at locations that will not affect the gate's ability to slant against the distal mating edges.

A sealing member can be provided on the interior surface of the gate.

The sealing member can embody an elastic member.

The sealing member can be made from a soft rubber or silicone material.

The sealing member can be attached to the first arm by forming two or more tabs on the first arm that can be shaped or folded to grip the sealing member.

In one example, slits or cuts can be formed through the first arm that can then be folded to grab the sealing member.

In other examples, adhesive or bonding can be used with or without the folded tabs to secure the sealing member to the first arm.

The sealing member can be sized and shaped and have a sufficient thickness to cover the needle bevel as the gate folds over the needle tip.

An extension can be provided at a distal end of the first arm for securing the gate to the second arm when the needle guard is activated to a protective position.

Activation of the needle guard can be carried out using one of several methods discussed elsewhere herein.

The extension can be straight, can have a hook, can include detents, can include an adhesive, or combinations thereof.

The extension on the first arm can be configured, such as sized and shaped, to secure against a second extension or a mating surface on the second arm.

In some examples, the needle guard can include additional sealing members located in the needle holding chamber of the needle guard, which can be understood to include a space between the two arms and the sidewalls for accommodating the needle.

The additional sealing members can further assist with sealing a chamber around the needle tip and the needle bevel following activation of the needle guard over the needle tip.

In some examples, sensors can be added to the needle guard.

In one example, a strain gauge can be included to detect or sense activation of the needle guard.

When the first arm, the second arm, or both the first arm and the second arm are bent or coiled, the strain gauge can trigger a signal to notify that the needle guard has been activated.

An open loop circuit can also be provided to sense when the first arm and the second arm contact one another, which closes the circuit when the two contact to trigger a signal to notify that the needle guard has been activated as the first arm now contacts the second arm to close the circuit.

A further aspect of the present disclosure includes a needle device, which can comprise a first hub having a body defining an interior cavity and having a proximal opening, a catheter tube extending distally of the first hub and comprising a distal opening, a second hub, a needle extending distally of the second hub, said needle comprising a shaft, a needle tip extending distally of the distal opening of the catheter tube in a ready to use position, and a change in profile located along the shaft, and a needle guard comprising a proximal wall having a perimeter defining an opening for the needle to pass therethrough, a first arm extending distal the proximal wall, a second arm extending distal the proximal wall opposite the first arm, the first arm and the second arm spaced from the needle in the ready to use position, the first arm and the second arm covering the needle tip in a protective position when the first arm and the second arm is triggered by an activator.

The needle guard can be formed by manipulating and folding a single patterned sheet of metal or other material.

The other material can be a composite material.

The composite material can be a carbon fiber composite.

The activator can be press fitted against the interior cavity.

The activator can be inside the interior cavity and prevented from moving proximally by a projection extending from the interior cavity.

The projection can be an annular ring.

The activator can extend radially inward.

The activator can be a tapered bore.

The activator can have a rectangular bore.

The activator can have a tapered proximal face.

The activator can have a tapered distal face.

The activator can have a cylindrical outer surface.

The activator can have a tapered outer surface.

The first flap and the second flap can have one or more activating portions, the one or more activating portions of the first arm having an arcuate surface along respective widths of the first arm extending distally in a first configuration, the one or more activating portions of the second arm having an arcuate surface along respective widths of the second arm extending distally in a first configuration.

The arcuate surface can be concave.

The arcuate surface can be convex.

The one or more activating portions of the first flap and the second flap can coil towards the needle in a second configuration to cover the needle tip in the protective position.

The activating portion of the first flap and the second flap can have a substantially flat surface in the protective position. In other examples, the activating portion of the first flap and the second flap can have a slight curve or bend.

The top flap can be longer than the bottom flap.

A distal extension can extend distally from the top flap.

The distal extension can be parallel to the top flap.

The distal extension can be bent relative to the top flap.

The distal extension can engage the bottom flap in the protective position.

One or more sidewalls can extend from opposite sides of the top flap.

One or more sidewalls can extend from opposite sides of the bottom flap.

Gaps can be provided between the one or more sidewalls.

One or more activating portions are located at regions without sidewalls.

Another aspect of the present disclosure can include a method of forming a needle device. The method can comprise providing a needle device comprising a first hub having a body defining an interior cavity and having a proximal opening, a catheter tube extending distally of the first hub and comprising a distal opening, a second hub, a needle extending distally of the second hub, said needle comprising a shaft, a needle tip extending distally of the distal opening of the catheter tube in a ready to use position, and a change in profile located along the shaft, and a needle guard comprising a proximal wall having a perimeter defining an opening for the needle to pass therethrough, a first arm extending distal the proximal wall, a second arm extending distal the proximal wall opposite the first arm, the first arm and the second arm spaced from the needle in the ready to use position, the first arm and the second arm covering the needle tip in a protective position when the first arm and the second arm is triggered by an activator.

The needle guard can be formed by manipulating and folding a single patterned sheet of metal or other material.

When activated to the coil configuration, the width of the first flap, second flap, or both flattens out, or at least have less of a curve, less of a bend, or less of an arc shape than when in the extended configuration. The length of each flap can coil up just one flap can coil up, or just the hinged section can coil up following activation.

The other material can be a composite material.

The composite material can be a carbon fiber composite.

The activator can be press fitted against the interior cavity.

The activator can be inside the interior cavity and prevented from moving proximally by a projection extending from the interior cavity.

The projection can be an annular ring.

The activator can extend radially inward.

The activator can be a tapered bore.

The activator can have a rectangular bore.

The activator can have a tapered proximal face.

The activator can have a tapered distal face.

The activator can have a cylindrical outer surface.

The activator can have a tapered outer surface.

The first flap and the second flap can have one or more activating portions, the one or more activating portions of the first arm having an arcuate surface along respective widths of the first arm extending distally in a first configuration, the one or more activating portions of the second arm having an arcuate surface along respective widths of the second arm extending distally in a first configuration.

The arcuate surface can be concave.

The arcuate surface can be convex.

The one or more activating portions of the first flap and the second flap can coil towards the needle in a second configuration to cover the needle tip in the protective position.

The activating portion of the first flap and the second flap can have a substantially flat surface in the protective position.

The top flap can be longer than the bottom flap.

A distal extension can extend distally from the top flap.

The distal extension can be parallel to the top flap.

The distal extension can be bent relative to the top flap.

The distal extension can engage the bottom flap in the protective position.

One or more sidewalls can extend from opposite sides of the top flap.

One or more sidewalls can extend from opposite sides of the bottom flap.

Gaps can be provided between the one or more sidewalls.

One or more activating portions are located at regions without sidewalls.

Yet another aspect of the present disclosure can include a method of using a needle device. The method can comprise providing a needle device comprising a first hub having a body defining an interior cavity and having a proximal opening, a catheter tube extending distally of the first hub and comprising a distal opening, a second hub, a needle extending distally of the second hub, said needle comprising a shaft, a needle tip extending distally of the distal opening of the catheter tube in a ready to use position, and a change in profile located along the shaft, and a needle guard comprising a proximal wall having a perimeter defining an opening for the needle to pass therethrough, a first arm extending distal the proximal wall, a second arm extending distal the proximal wall opposite the first arm, the first arm and the second arm spaced from the needle in the ready to use position, the first arm and the second arm covering the needle tip in a protective position when the first arm and the second arm is triggered by an activator; retracting the second hub following successful venipuncture; engaging the change in profile with the opening of the proximal wall; and triggering the first arm and the second arm to coil towards the needle by engaging the first arm and the second arm with the activator.

A still further aspect of the present disclosure is a needle device, comprising: a first hub having a body defining an interior cavity and having a proximal opening; a catheter tube extending distally of the first hub and comprising a distal opening; a second hub; a needle extending distally of the second hub and through said first hub and said catheter tube in a ready to use position, said needle comprising a shaft, a needle tip extending distally of the distal opening of the catheter tube, and a change in profile proximal of the needle tip; a needle guard comprising a proximal wall having a perimeter defining an opening having the needle passing therethrough, a first arm extending distally of the proximal wall and a second arm extending distally of the proximal wall opposite the first arm; and wherein the first arm and the second arm are spaced from the needle in the ready to use position and the needle guard is in a first configuration, and wherein the first arm and the second arm cover the needle tip in a protective position when the first arm and the second arm are activated and part of a width of the first arm, the second arm, or both change from a first shape to a second shape having less of a curved surface and the needle guard is in a second configuration.

The needle device can further comprise an activator for activating the first and second arms.

The activator can be pressed fitted into the interior cavity.

The activator can be located inside the interior cavity and be prevented from moving proximally by a projection extending from an interior surface of the interior cavity.

The projection can be an annular ring inside the first hub.

The projection can comprise a single bump or stub extending from an interior surface of the first hub.

The needle guard can undergo a phase change from an extended state or first configuration to a coiled state or second configuration. When undergoing a phase change, certain dimensions of the needle guard, such as the width of the flap or of both flaps, which is measured orthogonally to the lengthwise axis of the needle guard, can change from a curved profile to one that is less curved. In some example, the les curved profile comprises a generally flat profile along the width.

The activator can extend radially inward from an interior surface.

The activator can have a tapered bore.

The activator can have a rectangular bore.

The activator can have a tapered proximal face.

The activator can have a tapered distal face.

The activator can have a cylindrical outer surface.

The activator can have a tapered outer surface.

The first shape of the first flap and the second flap can comprise an arcuate surface formed along one or more activating portions.

The arcuate surface can be concave.

The arcuate surface can be convex.

The one or more activating portions of the first flap and the second flap can coil towards the needle in the second configuration to cover the needle tip.

The second shape of the first flap and the second flap can comprise a generally flat surface.

The first flap can be longer than the second flap.

A distal extension can extend distally from the first flap.

The distal extension can extend parallel to the first flap.

The distal extension can include a bend relative to the first flap.

The distal extension can engage the second flap in the protective position.

One or more sidewalls can extend from opposite sides of the first flap.

Gaps can be provided between the one or more sidewalls.

One or more activating portions on the needle guard can be located at regions without sidewalls.

A method of forming a needle device, the method can comprise obtaining a bistable metal, stamping or cutting sections and/or regions from the bistable metal to produce a foldable body, and bending or manipulating the foldable body to produce a needle guard as described herein.

A method of using a needle device, the method can comprise: providing a needle device as described herein; retracting a second hub following successful venipuncture; engaging a change in profile on a needle with an opening on a proximal wall of the needle guard; triggering a first arm and a second arm of the needle guard to coil the arms towards the needle by subjecting the first arm and the second arm to a force sufficient to force the first and the second arm to undergo a phase change from an extended configuration to a coiled configuration.

In some examples, the force on the needle guard can be generated by an activator located inside a first hub.

The sidewalls on the flaps of the needle guard can have similar or different shapes and can have a gap between two adjacent sidewall sections.

In the ready to use position, the flaps can be spaced from the needle.

In the ready to use position, the flaps can be spaced from the interior wall surfaces of a first hub.

The first hub can be a catheter hub. The second hub can be a needle hub.

In other examples, the name designation can be reversed. For example, a first hub can be a needle hub and the second hub can be a different hub, such as a catheter hub. The terms first and second are intended to identify two different structures and not structurally limiting unless the context indicates otherwise.

Broadly speaking, the present disclosure is directed to a needle assembly having a first hub with a needle guard having bistable configurations, phases, or states located therein mounted on a needle extending from a second hub. The needle guard can be activated to change state from a first or extended state to a second or coiled state. In the coil state, the needle guard can cover the needle tip of the needle. The needle guard can change state from the first state to the second state by using an activator to activate the transition. An activator can be located inside the first hub to activate the needle guard. The activator can be unitarily formed with the first hub or separately formed and subsequently assembled to the first hub. Activation can occur by moving the needle and the needle guard relative to one another. The activator can embodiment a physical structure that physically contacts the needle guard to cause the needle guard to change state from a first or extended state to a second or coiled state. Movement relative to one another can permit a change in profile on the needle to abut a perimeter with an opening on a proximal wall of the needle guard. The abutment can force the needle guard and an activator inside a first housing to interact to cause the needle guard to change state from a first or extended state to a second or coiled state.

The first hub can be a housing without a catheter tube or can be a catheter hub having a catheter tube.

Relative movement between a needle guard and a needle can be effected by gripping a first hub having the needle guard located therein and retracting a second hub attached to the needle away from the first hub. Both the first hub and the second hub can simultaneously move away from one another, the first hub can remain steady or stationary while the second hub moves away from the first hub, or the second hub can remain steady or stationary while the first hub moves away from the second hub. In other examples, the first hub can be held by something other than a user or practitioner's hands. For example, the first hub can be secured by a tether or an adhesive pad. The tether has two ends with one end connected to the first hub and the second end to a different component. In some examples, the different component can be a strap, an adhesive pad or strip, or to another or different needle assembly.

A needle assembly having a needle guard with bistable configurations, phases, or states can be a fistula needle assembly, a catheter assembly, or any needle assembly having a first hub with the needle guard located therein and a second needle hub having a needle extending therefrom. The first hub can have features discussed elsewhere herein. The second hub can have features discussed elsewhere herein.

Another aspect of the present disclosure is a needle device comprising a first hub having a body defining an interior cavity and having a proximal opening; a second hub; a needle extending distally of the second hub and through the first hub in a ready to use position, said needle comprising a shaft, a needle tip, and a change in profile proximal of the needle tip; a needle guard located in the interior cavity of the first hub, said needle guard comprising a proximal wall having a perimeter defining an opening having the needle passing therethrough, a first arm extending distally of the proximal wall and having a width and a second arm extending distally of the proximal wall opposite the first arm and having a width; and wherein the first arm and the second arm are spaced from the needle in the ready to use position and the needle guard is in a first configuration, and wherein at least the first arm covers the needle tip in a protective position and the needle guard is in a second configuration when the needle guard is activated and part of the width of the first arm, the second arm, or both change from a first shape to a second shape having less of a curved surface.

The needle device can further comprise an activator for activating the needle guard to change from the first configuration to the second configuration. The activator can be located inside the first hub.

The first hub can be a catheter hub comprising a catheter tube. The first hub can further comprise a seal having one or more slits for sealing the interior cavity of the catheter hub. The seal can completely stop or reduce blood flow into the interior cavity of the catheter hub.

The needle can be a fistula needle.

The needle device can further comprise a sealing member located on an interior surface of the first arm for sealing a bevel of the needle tip.

The needle device can further comprise a tether having a first end attached to the first hub.

A second end of the tether can attach to a needle hub of a second needle assembly, such as an arterial needle assembly.

The second end of the tether can alternatively be attached to a strap, a band, an adhesive pad, adhesive strip, or combinations thereof.

### BRIEF DESCRIPTION OF THE DRAWINGS

These and other features and advantages of the present devices, systems, and methods will become appreciated as the same becomes better understood with reference to the specification, claims and appended drawings wherein:
FIG. 1A is a cross-sectional side view of a needle device with a needle guard in a ready to use position;
FIG. 1B is a sectional end-view taken along line 1B-1B of FIG. 1A, with the needle removed for clarity;
FIG. 2A is a plan view of the needle guard in an unfolded state, showing the body before folding;
FIG. 2B is an end view of the body of FIG. 2A in an extended configuration showing an arc-shaped width;
FIG. 2C is a side view of the body of FIG. 2A in a coiled configuration;
FIG. 3A is a close-up side view of the needle guard in the needle device of FIG. 1A with the needle retracted from the ready to use position and before the needle tip enters the needle guard;
FIG. 3B is a perspective view of the needle device of FIG. 3A with the needle guard retracted from the ready to use position;
FIG. 4A is a front view of the needle device in a protective position; and
FIG. 4B is a perspective view of the needle device of FIG. 4A in the protective position.
FIG. 5 is a cross-sectional side view of a fistula needle provided in accordance with aspects of the present disclosure.
FIGs. 6A-6C show different modes or configurations for activating a needle guard.
FIG. 7A and 7B are schematic drawings showing the needle guard of the present disclosure usable to contain or seal off a needle bevel to restrict or limit fluid flow.

### DETAILED DESCRIPTION

The detailed description set forth below in connection with the appended drawings is intended as a description of the presently preferred embodiments of needle devices and their components provided in accordance with aspects of the present assemblies, systems, and methods and is not intended to represent the only forms in which the present devices, systems, and methods may be constructed or utilized. The description sets forth the features and the steps for constructing and using the embodiments of the present assemblies, systems, and methods in connection with the illustrated embodiments. It is to be understood, however, that the same or equivalent functions and structures may be accomplished by different embodiments that are also intended to be encompassed within the spirit and scope of the present disclosure. As denoted elsewhere herein, like element numbers are intended to indicate like or similar elements or features.

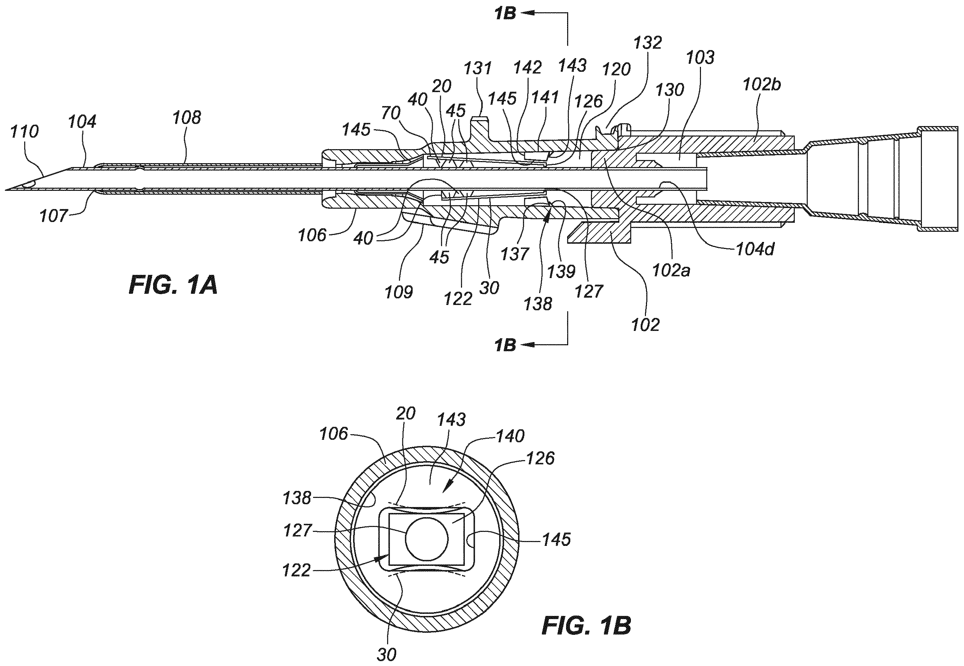

With reference now to FIG. 1A, a cross-sectional side view of an embodiment of a needle device 100, such as an over-the-needle catheter assembly or an IV catheter assembly, provided in accordance with aspects of the present disclosure is shown. FIG. 1A shows the needle device 100 in an installed or ready to use position in which a needle tip 110 of a needle 104 extends distally of a distal end 107 of the catheter tube 108 for accessing the vasculature of a patient. In an example, the needle device 100 comprises a first hub 106 having a hub body with a proximal end 130, an interior cavity 120, a catheter tube 108 extending distally from a distal end of the catheter hub 106, a second hub 102 attached to the proximal end 130 and having a cannula or needle 104 extending distally of the needle hub 102 and projecting through the proximal end 130 and the catheter tube 108 and having the needle tip 110 extending distally of a distal opening of the catheter tube 108. The first hub can be a catheter hub and the second hub can be a needle hub. A needle guard or spring clip 122 is slidably positioned on the needle 104 and can be located in the interior cavity 120 of the catheter hub 106 in the ready to use position. A trigger or activator 140 can be located in the interior cavity 120 distal of the Luer taper of the catheter hub 106. A proximal end of the catheter tube 108 can attach to the catheter hub 106 using a bushing 144 or other conventional means. As used herein, the term distal means an end closer to the needle tip 110 and the term proximal is the end farther away.

In the ready position, the needle 104 projects distally from a nose section 102a of the needle hub 102 through the catheter hub 106. The needle 104 has a needle tip 110 extending out a distal end 107 of the catheter tube 108, as previously described. A proximal end of the needle 104 can extend into a flashback chamber 103 of the needle hub 102, which has an opening at a proximal end 102b that can be closed by a vent plug 133. Optionally, the vent plug can have sampling features that allow blood to be collected and dispensed, such as on a glucose strip or a Petri disc. An exemplary blood stopper can be similar to the blood stopper disclosed in pending U.S. Patent Application No. 14/576,802, filed December 19, 2014, the contents of which are expressly incorporated herein by reference.

The needle tip 110 is configured for penetrating the epidermal layer of a patient and accessing the vasculature of the patient. The needle hub 102 can have a needle guard extension 125 extending from the nose section 102a into the open chamber 120b of the interior cavity 120 for aligning the needle guard 122 during assembly, the details of which are further described below.

The proximal end 130 of the catheter hub 106 can have a female Luer taper with external threads 132, also known as a threaded female Luer connector. The female Luer connector is configured to matingly receive a male Luer connector, such as an IV line, a Luer access connector, a syringe tip, a vent plug, an IV set, an extension set, another known connector, or future-developed IV devices with a Luer tip. Each of these components can be sized and configured in conformity with at least some of the International Standards Organization (ISO) standards for female and male Luer connections under current or future standards. For discussion purposes, any one of these components or the class of these components can be referred to as a male medical implement or a male connector. As shown, the threaded female Luer connector receives a nose section 102a of the needle hub 102. A physical abutment is provided between the catheter hub 106 and the needle hub 102 so as to set the distance the needle tip extends out the distal end 107 of the catheter tube.

The catheter hub 106 can include a tab 131 on an exterior thereof configured for use as leverage when handling the device 100, such as to push against during insertion or removal of the needle 104. A pair of wings 109 may extend laterally of the catheter hub 106 to support and stabilize the catheter hub 106 against the patient following successful venipuncture. Optionally, the tab 131 and the wings 109 can be omitted.

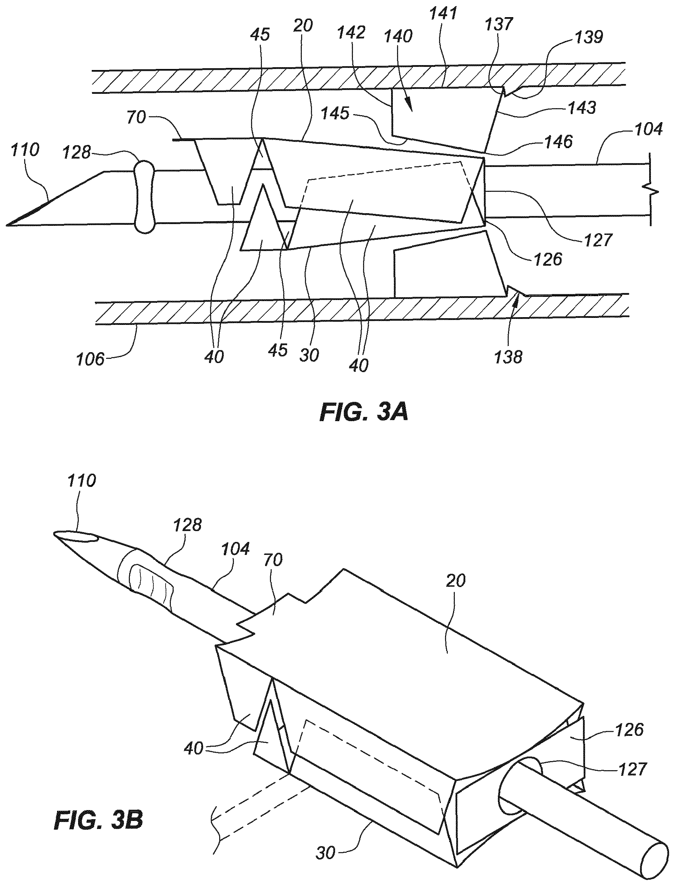

A trigger or activator 140 can be incorporated for triggering the needle guard 122 to change its shape from an extended structural configuration to a coiled structural configuration to cover the needle tip 110 in a protective position, when the needle guard 122 is withdrawn proximally from the ready to use position after successful venipuncture. The trigger 140 can be unitarily formed with the catheter hub or separately formed and subsequently attached to the catheter hub. Explanations of both structural configurations of the needle guard 122 and the trigger 140 are further discussed below. In an example, when the needle guard 122 is in an extended structural configuration, such as that shown in FIGs. 1A and 3A, the distal ends of the two arms are spaced from the needle shaft so that no drag is exerted on the shaft by the flaps or arms 20, 30 of the needle guard 122 when the needle 104 moves relative to the needle guard, such as following placement of the catheter tube in a patient's vasculature. In other examples, no part of the two arms contacts the shaft in the extended structural configuration.

The activator 140 can be located in the interior cavity 120 by pressing a generally cylindrical outer surface 141 of the activator against the interior wall surfaces of the interior cavity 120, distal of the female Luer chamber of the catheter hub. The shape or contour of the outer surface 141 can match the surface of the interior cavity 120, such as being straight or taper. In one example, the outer surface 141 can include surface features (not shown) to align the activator 140 in a particular orientation inside the interior cavity 120. For example, a notch and a groove can be incorporated between the activator and the interior cavity to facilitate orienting the activator inside the catheter hub.

A projection 138, which can act as a stop, can be included inside the interior cavity 120 to prevent the activator 140 from displacing proximally out of position from a set position inside the interior cavity 120. The projection 138 can embody a continuous protrusion formed around a periphery or interior diameter of the interior cavity 120. In another example, the projection 138 can comprise a single bump or two or more spaced apart sections formed around a periphery of the interior cavity 120 instead of a continuous annular ring. The projection 138 can have two surfaces forming an apex wherein the two surfaces can include a blocking surface 137 and an inclined surface 139 proximal of the blocking surface 137. The inclined surface 139 elastically deforms the activator 140 as the activator 140 is pushed distally inside the interior cavity 120 over the inclined surface 139 and past the apex. Once the activator 140 has cleared the apex, the activator 140 can snap back into its original shape with the proximal end of the activator 140 contacting the blocking surface 137. The blocking surface 137 can prevent the activator 140 from moving proximally past the blocking surface 137 when the needle guard is retracted proximally during retraction of the needle following successful venipuncture. Alternatively, the activator 140 can be press fitted inside the interior cavity 120 thus eliminating the need for the projection 138. In other examples, the activator can be pressed fit inside the catheter hub and a projection 138 is also incorporated.

The activator 140 can be made from a number of materials, including from plastic or metal. The activator 140 can have a distal face 142 at a distal end, a proximal face 143 at a proximal end, and a bore 145 extending along a lengthwise axis of the activator through the distal face 142 and the proximal face 143. The distal face 142 can be flat or have a conical shape which tapers inwardly or outwardly. The proximal face 143 can also be flat or have a conical shape which tapers inwardly so as not to interfere with a male Luer taper inserted into the Luer chamber through the proximal opening 130. The shapes of the distal face 142 and proximal face 143 are not limited and can take on a variety of shapes to serve any purpose. In other examples, the activator 140 can be hollow and/or made from a resilient material that readily deforms when pushed into the bore of the catheter hub, such as from a polymer material.

FIG. 1B illustrates an end-view of the needle device 100 taken from line 1B-1B of FIG. 1A, with the needle 104 removed for clarity. The bore 145 of the activator 140 can be sized and shaped to activate or trigger the needle guard 122 from the extended configuration to the coiled configuration and to allow the needle guard 122 and the needle 104, including the needle tip 110, to pass through the bore 145 in the proximal direction when the needle guard 122 is in the protective position blocking the needle tip. When the needle guard changes from the extended configuration to the coiled configuration or when the needle guard changes from coiled configuration back to the extended configuration, the needle guard can be said to undergo a change in state or a phase change. This term is used differently than its normal meaning, which typically means a change between gas, liquid, and solid phases.

In one example, the needle guard 122 in the extended configuration cannot pass through the bore 145 of the activator without contacting the activator 140. As shown, the bore 145 can be rectangular in shape and have a length that tapers inwardly from the distal face 142 in the proximal direction. The interior surfaces of the bore 145 can be flat, concave, or convex. In other examples, the bore 145 can be generally cylindrical with a taper lip at the entrance. The bore 145 can be sized and shaped to activate the needle guard 122 and to allow the needle guard to pass from a distal position of the activator 140 to a proximal position of the activator, such as following successful venipuncture and as the needle 104 is retracted away from the catheter hub 106 with the needle guard 122 covering the needle tip 110.

Activating edges 146 formed between a surface of the bore 145 and the proximal face 143 of the activator can be configured to engage and trigger the needle guard 122 to change its configuration as the needle guard 122 is withdrawn proximally. The activating edges 146 can be flat, concave, or convex. The activating edges can be located along the same axial position of the activator 140 or along different axial positions of the activator 140 to contact and activate the needle guard at different time and/or different locations of the needle guard. In other examples, other surfaces and features of the bore 145 or activator 140 can be configured to change the configuration of the needle guard 122. For example, the needle guard 122 can contact a surface of the bore 145 other than the proximal activating edges 146 to change the configuration of the needle guard 122 from the extended structural configuration to the coiled structural configuration. Details of the interaction between the activator 140 and the needle guard 122 and how the needle guard changes its structural configuration are discussed below.

As shown in FIGs. 1A, 1B, and 3A, the needle guard 122 is positioned completely inside the interior cavity 120 of the catheter hub 106 in the ready position and during needle withdrawal prior to securing the needle tip 110 with the needle guard 122 in the protective position. In other examples, the needle guard 122 can be positioned partially inside the catheter hub body 106 or completely outside the catheter hub 106, such as in a shroud or a separate needle guard housing between the catheter hub 106 and the needle hub 102, so long as the needle guard 122 can be activated to a coiled position by the activator 140 to prevent exposure of the needle tip 110.

In an example, the needle guard 122 can comprise a proximal wall 126 and two flaps 20, 30, such as a first or top flap 20 and a second or bottom flap 30, extending distally of the proximal wall 126. The flaps 20, 30 can have the same width or different widths and the same length or different lengths. The flaps 20, 30 may alternatively be labeled or called arms. Optionally, a distal extension or extension 70 can extend from a distal end of the top flap 20 or on an end opposite the proximal wall 126. The extension 70 can also extend from the side flaps 40. The distal extension 70 can include one or more bent sections extending towards the needle 140 or remain parallel with the top flap 20. In another example, a second distal extension can extend from a distal end of each of the two flaps 20, 30 or from the side flaps of both the upper and lower arms. The flaps 20, 30 of the needle guard can be spaced from the interior wall surfaces of the interior cavity 120, the needle 104, and the activator 140 in the ready to use position. During needle retraction, the needle guard 122 can slide proximally and contact the activator 140 but not actuated by the activator 140 until after being pulled in the proximal direction by the change in profile on the needle 104 and into the activator 140. In other examples, during retraction, the needle guard 122 can be spaced from the activator 140 and not touch the activator 140 so that the needle 104 can move within the opening on the proximal wall 126 until the change in profile on the needle 104 engages the proximal wall 126. The length of the needle guard 122 can be greater than a distance between the change in profile 128 and the needle tip 110 to ensure capturing of the needle tip 110 in the needle guard 122 in the protective position.

The proximal wall 126 can comprise a perimeter defining a proximal opening 127 having the needle 104 passing therethrough. The perimeter of the proximal opening 127 is configured for engaging the change in profile 128 of the needle 104 following placement of the catheter tube 108 into a patient's vasculature. The change in profile 128 can be a crimp, a bulge, a sleeve, or a material buildup formed on the shaft of the needle 104 adjacent the needle tip 110 to prevent the needle guard 122 from displacing distally off of the needle 104 in the protective position.

In one example, the length of one of the flaps 20, 30 can be greater than the length of the other of the flaps 20, 30. The widths of the two flaps can be the same or different. Having different lengths can allow the two flaps 20, 30 to stagger when in the coiled configuration, as further discussed below. In another example, the length of the two flaps 20, 30 can be substantially the same. Each of the top flap 20 and the bottom flap 30 can have one or more sidewalls 40 extending from opposite sides of the top flap 20 and the bottom flap 30. The one or more sidewalls 40 can extend towards each other. The length, width, and shape of the sidewalls 40 can be uniform or can vary. The two flaps 20, 30, the proximal wall 126 and the sidewalls 40 of the needle guard 122 can define a needle tip holding space when the needle tip 110 is located therein, such as when the needle guard 122 is activated and covers the needle tip 110. The sidewalls 40 can prevent the needle 104 from moving sideways out of the sides of the needle guard 122 and out of the needle holding space.

The sidewalls 40 can increase the structural rigidity of the flaps 20, 30. Along a side of each flap 20, 30, a gap 45 can be provided between two adjacent sidewalls 40. The gap 45 can be trapezoidal (FIG. 1A), triangular (FIG. 3), rectangular, or other shapes that provide sufficient space or room for the sidewalls 40 to move when the flaps 20, 30 are activated, as further discussed below. Thus, the dimension and shape of each gap 45 is not limited to triangles and trapezoids or other shapes and can vary depending on the shape of the adjacent sidewalls 40 and the extent or amount of movement of the flaps 20, 30 following activation. At least one of the top flap 20 and the bottom flap 30 can have an arcuate surface along their respective widths. In other words, if each flap 20, 30 has a length along the lengthwise axis of the needle 104 and a wide orthogonal thereto, the side has an arcuate surface. In some examples, the entire width is formed along an arc or a curve. In some examples, the entire width of one or both flaps can be flat or not curved.

As shown, both the top flap 20 and the bottom flap 30 have an arcuate surface along the width. The arcuate surface of the top flap 20 and the bottom flap 30 can be concave or convex and can extend the entire length of the flap distal of the proximal wall 126. The wall surface of the flaps 20, 30 can have a substantially constant thickness and the arcuate surface can have a substantially constant radius of curvature or can have a complex curve. In other examples, the flaps 20, 30 can have a variable thickness and the radius of curvature of the arcuate surface can vary along the width. The arcuate surface of the flaps 20, 30 can force the flaps 20, 30 to extend distally in the extended structural configuration along a lengthwise direction. In other words, by maintaining an arc along the width for the length of the flap 20, 30, the arcuate surface can make the flaps 20, 30 stable in the extended configuration. The arc-shape structure provides reinforcement and stiffness that allow the flap to extend axially without breaking, collapsing, distorting, buckling or otherwise undergo a phase change until activated by a sufficient force, such as by an activator. The sidewalls 40 along the lengthwise direction of the flaps 20, 30 can stiffen the flaps 20, 30 by increasing the moment of inertia. The presence of the sidewalls 40 can prevent portions of the flaps 20, 30 from transitioning into the coiled configuration.

It has been found that when gaps 45 are provided in the sidewalls 40 along the lengthwise direction of each flap 20, 30, the structural rigidity of the flaps 20, 30 along the activating portions can be altered between the extended configuration and the coiled configuration. This information can be used to control or select the activating portions of the flaps 20, 30 to transition to a coiled configuration when triggered by the activator 140. In some examples, sections of the flaps 20, 30 are not rolled to not create a bi-stable situation. This can be incorporated so that sections of the flaps 20, 30, even without the side walls 40, can remain straight and not coil when activated. Thus, the location and shape of the sidewalls 40 and gaps 45 on the flaps 20, 30 can control the shape of the needle guard 122 in the coiled configuration and therefore how the flaps 20, 30 in the coiled configuration can cover the needle tip 110 in the protective position. Details of the mechanics of the flaps 20, 30 of the needle guard 122 morphing from an extended structural configuration to a coiled structural configuration are illustrated below with reference to an unfolded state of a needle guard, prior to folding the body into its final needle guard shape. The structure that can morph between one stable structural configuration or state to another stable structural configuration or state is known as a bistable structure or a structure with bistable phases.

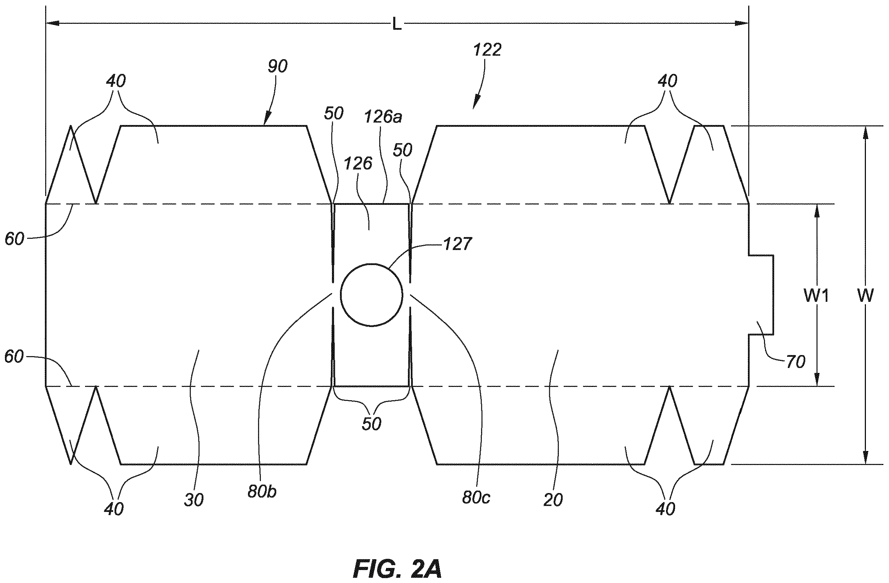

Referring now to FIGs. 2A-2C, the needle guard 122 can be formed from a body 90 having an arcuate surface along a width or along a width section, as shown with notation "W," which is generally orthogonal to the length. The body 90 can be stamped or cut in the unfolded state from a length of metal sheet or other material having an arcuate surface along the width. The body 90 and the various cut-out sections of the body 90 can be folded or manipulated to form various sections of the needle guard 122. In one example, the body 90 is generally rectangular having a length L, a width W extending orthogonally of the length, a substantially constant thickness T, and an arcuate surface along the width. The arcuate surface can have a substantially constant radius of curvature R along the width or can have a complex curve and the shape can extend along the lengthwise direction of the body.

The arcuate shaped width of the body 90 has a concave surface 91 and a convex surface 93. Depending on the direction the body 90 is coiled in the lengthwise direction to create the second bistable state, the concave surface 91 can be located to the outside or the convex surface 93 can be located to the outside when the body is activated into its coiled configuration, as further discussed below.

Although the shape of the body 90 is shown as being generally rectangular, the shape of the body 90 is not limited to the generally rectangular shape and in other examples the shape of the body 90 can have a regular or irregular polygonal shape or can comprise multiple pieces that can be attached or pieced together. In still other examples, the thickness T of the body 90 is not limited to a constant value and can vary. The radius of curvature R is also not limited to a constant value and can vary along the widthwise direction, such as having a complex curve.

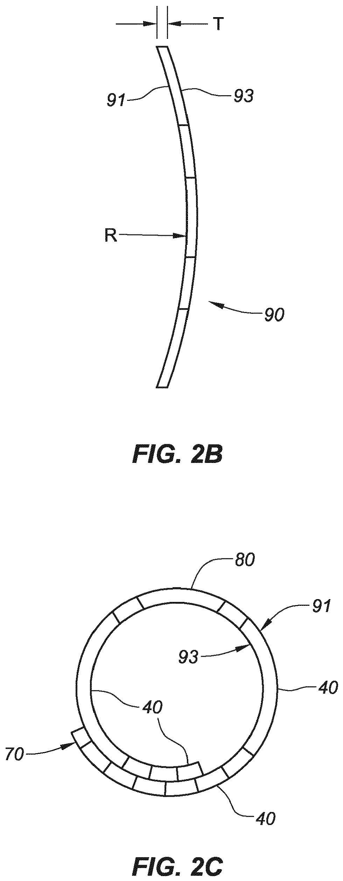

A unique aspect of the body 90 is that one or more sections of the body can be a bistable structure, which means that the one or more sections of the body are capable of morphing between two stable structural configurations. It also means that at least a width section of a flap or both flaps can alternate from a first shape to a second shape and wherein the first shape is characterized by a curved surface and the second state is characterized by a surface that is less curved, such as being more flat or generally flat. As a point of reference, an example of a bistable structure is a slap bracelet, which is widely commercially available as kids' toys and reflectors for cyclists. The two stable configurations can be stabilized by different curvature axes. A first stable configuration of the two stable mechanical configurations can be the extended configuration and the second stable configuration can be the coiled configuration. In the extended configuration, the arcuate surface of the body 90 forces the body 90 in a straight extended configuration along the lengthwise direction, as discussed above. That is, the arcuate surface of the body 90 forces the body 90 to be stable in the extended configuration. An example of the extended configuration is shown in FIGs. 2A and 2B.

The body 90 can remain in the extended configuration until an external force is applied to the body 90, which then transitions the body 90 from the first stable configuration (extended configuration) to the second stable configuration (coiled configuration). For example, a force, such as an impulse force, can be applied to flatten or bend any part of the concave cross-section of the body 90 to change the body into its coiled configuration. To add, in the extended configuration, the width of the flap can have a bend or arc section. However, when activated to the coil configuration, the width flattens out, or at least has less of a curve, less of a bend, or less of an arc shape than when in the extended configuration, as the length of the body coils up to the second stable configuration. Once in transition, the body 90 can continue transitioning from the extended configuration to the coiled configuration without additional force because internal stresses in the body cause the body 90 to morph from the extended configuration into the coiled configuration. During the transition, the arcuate surface of the body 90 flattens thereby forcing the body 90 to curve or coil along an axis different than the axis of the arcuate surface. The coiling axis can be perpendicular to the arcuate surface axis.

Thus, an aspect of the present disclosure is understood include a needle device, comprising: a catheter hub having a body defining an interior cavity and having a proximal opening; a catheter tube extending distally of the catheter hub and comprising a distal opening; a needle hub; a needle extending distally of the needle hub and through said catheter hub and said catheter tube in a ready to use position, said needle comprising a shaft, a needle tip extending distally of the distal opening of the catheter tube, and a change in profile proximal of the needle tip; a needle guard comprising a proximal wall having a perimeter defining an opening having the needle passing therethrough, a first arm extending distally of the proximal wall and a second arm extending distally of the proximal wall opposite the first arm; and wherein the first arm and the second arm are spaced from the needle in the ready to use position and the needle guard is in a first configuration, and wherein the first arm and the second arm cover the needle tip in a protective position when the first arm and the second arm are activated and part of a width of the first arm, the second arm, or both change from a first shape to a second shape having less of a curved surface and the needle guard is in a second configuration. In some examples, the needle guard is used with a hub with an interior cavity that is not a catheter hub.

An example of the body 90 in the coiled configuration is shown in FIG. 2C. In the coiled configuration, the body 90 is coiled along the lengthwise direction. The coiled body can be uniform or non-uniform in which certain sections coil more than others or where some sections are not coiled at all. Thus, in a coil stated, the body may not be circular or round but can have a polygonal shape or an irregular rolled up shaped. The body 90 is also stable in this coiled configuration. Thus, the body 90 cannot transition back to the first stable configuration unless an external force is applied to uncoil the body 90 back into the extended configuration. Once the body 90 returns to the extended configuration from the coiled configuration, the body 90 remains stable in the extended configuration until a force is again applied to the body 90, which then transitions the body back to the coiled configuration. Thus, the body 90 can transition between these two stable configurations, phases, or states.

In one example, the coiled configuration may be coiled in a coiling direction against or away from the arcuate surface. For example, as shown in FIG. 2C, the body 90 can be coiled such that when activated from the extended configuration to the coiled configuration, the concave surface 91 can be located to the exterior or outside of the coiled shaped structure. In another example, the coiled configuration may be coiled in the opposite direction such that the convex surface 93 is now located to the exterior of the coiled shaped structure when the body 90 is activated from the extended configuration to the coiled configuration.

In both examples of coiling the body 90 to create a coiled structure with either the concave surface 91 located on the exterior or the convex surface 93 on the exterior, the arcuate surface of the body 90, such as the width of the body, can flatten thus maintaining a substantially flat cross-section in the coiled configuration. The substantially flat cross-section combined with the coiling of the body 90 along an axis different to the axis of the arcuate surface allows the body 90 to be stable in the coiled configuration. In some examples, the shape can be less curved or can have less or a bend without completely or substantially flattened out.

Although the extended configuration and coiled configuration are described for the unfolded state of the needle guard 122, the same principles apply when the unfolded body 90 is folded into a shape of a needle guard, such as the needle guard shown in FIGs. 1A and 3A. Thus, the flaps or other sections of the needle guard 122 can have bistable configurations that allow the needle guard, such as one or both flaps or sidewalls, to be arranged in a ready to use position inside a catheter hub and a folded or coiled configuration to cover the needle tip following successful venipuncture. Further, while an exemplary application of the needle guard having sections that have different stable configurations of the present disclosure has been disclosed with a needle assembly, such as one with a catheter hub and a catheter tube, the needle guard may be used with any number of needle devices, with or without a catheter tube, such as fistula needles.

Referring again to FIG. 2A, the various sections of the body 90 can be manipulated, such as bent or curved, to include the proximal wall 126, the bottom flap 30 extending distally of a first connecting side 80b of the proximal wall 126, the top flap 20 extending distally of a second connecting side 80c of the proximal wall 126 opposite the first side, the optional distal extension 70 extending from the top flap 20, and one or more sidewalls 40 extending from opposite sides of the flaps 20, 30 with gaps 45 provided adjacent to or between one or more sidewalls 40. The portions of the flaps 20, 30 coinciding with the gaps 45 can be defined as activating portions, as discussed further below. The width or spacing of each gap and the number of gaps can be selected depending on the shape of the coiled configuration of one or more flaps, as desired. In other examples, the distal extension 70 can extend from other sections of the needle guard, such as from the bottom flap or from the one or more sidewalls.

In some examples, sidewalls 40 can be incorporated to prevent the needle tip 110 from protruding out a side of the flaps 20, 30 and stiffen the flaps 20, 30 at portions of the flaps 20, 30 bordered by the sidewalls. The portions of the flaps 20, 30 bordered by the sidewalls 40 can be defined as non-activating portions.

As shown in FIG. 2A, the portion of the body representing the proximal wall 80 can be generally rectangular with the connecting sides 80b, 80c each extending along a widthwise direction and having an arcuate cross-section. Because the connecting sides 80b, 80c have arcuate cross-sections, cutouts 50 can be provided extending inward from opposite sides of the connecting sides 80b, 80c to ease bending along the connecting sides 80b, 80c. As shown, there are four cutouts 50 located between the proximal wall 80 and the two flaps 20, 30. The cutouts 50 can form along upper and lower edges of the proximal wall 80, near the two connecting sides 80b, 80c. Folding lines 60 can be provided on the body 90 to designate foldable or desired locations when forming the needle guard 122. In one example, the folding lines 60 can be etched or marked on the body 90.

The needle guard 122 can be formed by folding the body along the folding lines 60. In one example, the body 90 can be folded with the arcuate surfaces of the top flap 20 and the bottom flap 30 of the needle guard 122 opening outwardly in a direction away from the needle 104. In another example, the body 90 can be folded with the arcuate surfaces of the top flap 20 and the bottom flap 30 of the needle guard 122 opening in a direction towards the needle 104. For example, in FIG. 1B, the arcuate surfaces of the flaps 20, 30 are concave with the curve facing outwardly in a direction away from the needle 104. Alternatively, in other embodiments, only the flaps 20, 30 have arcuate surfaces, and not the proximal wall. The separately formed flaps can be separately attached, directly or indirectly, to the proximal wall 126, such as by welding.

The one or more sidewalls 40 can be bent towards each other along the folding lines 60. The sidewalls 40 and the gaps 45 along the flaps 20, 30 can be arranged and shaped to ensure that when activating portions of the flaps 20, 30, the sidewalls 40 do not interfere with each other and obstruct the flaps' ability to coil, as discussed further below. The sidewalls 40 may also be bent outwardly or bent multiple times. The distal extension 70, if present, can be bent towards the bottom flap 30 or remain parallel with the top flap 20. The distal extension 70 can be incorporated for securing or locking the top flap 20 to the bottom flap 30 and/or to obstruct the needle tip from re-emerging out the distal end of the needle guard following activation thereof. After the needle guard 122 is formed, the needle guard 122 can be assembled onto the needle device 100 along with the activator and prior to assembling the needle to the needle hub. In other examples, the needle guard can be mounted onto the needle after the needle is attached to the needle hub. For example, the crimp or change in profile can be formed after the needle guard is slidably placed onto the needle.

Turning now to FIGs. 3A and 3B, the needle 104 can be retracted proximally away from the catheter hub 106 following successful venipuncture by pulling on the needle hub 102 in the proximal direction. Because the opening 127 on the proximal wall 126 is larger than the diameter of the shaft of the needle 104, the needle 104 can be retracted proximally without dragging or pulling on the needle guard 122 proximally from a needle guard set position or from the catheter hub. As the flaps 20, 30 of the needle guard are spaced from the needle in the ready to use position, the needle 104 can also be retracted following successful venipuncture without the needle shaft or the change in profile 128 contacting the flaps 20, 30. Thus, the needle guard 122 or the flaps 20, 30 can remain in the extended configuration while the needle 104 retracts in the proximal direction until the change in profile 128 engages the perimeter of the opening 127 in the proximal wall 126.

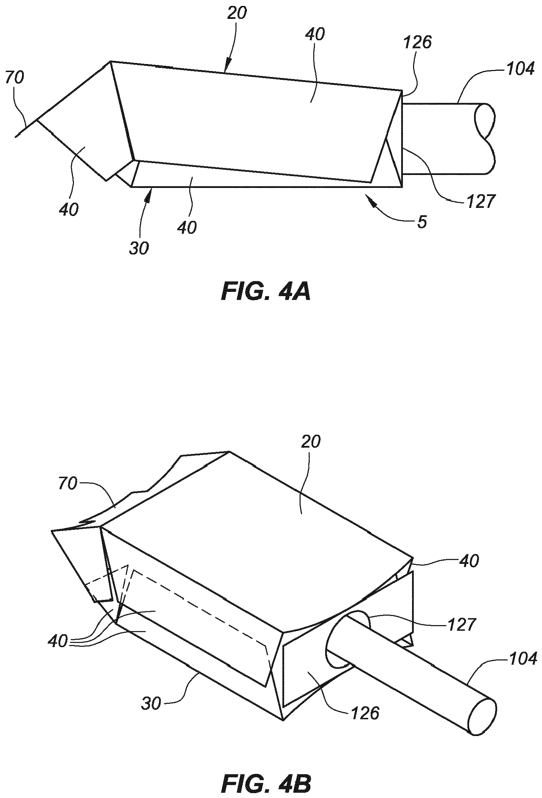

Referring now to FIGs. 4A and 4B, with the change in profile 128 engaged against the perimeter defining the opening 127, further retraction of the needle 104 can pull the needle guard 122 proximally towards activating edges 146 of the activator 140. When the flaps 20, 30 of the needle guard 122 engage the activating edges 146 or other surface features of the activator 140 and further movement of the needle guard 122 in the proximal direction against the resistance of the activator 140 continues, the contact forces experienced by the flaps 20, 30 will cause the flaps to move inward towards the needle 104. The contact forces can also cause the bistable nature of the flaps to undergo a change.

The flaps 20, 30 can transition from the extended configuration, as shown in FIGs. 1A, 3A, and 3B, to the coiled configuration to cover the needle tip 110, as shown in FIGs. 4A and 4B, when activated. Said differently, when the needle guard 122 is triggered, the activating portions of the flaps 20, 30 coil inwardly to cover the needle tip 110 in the protective position. That is, the activating portions of the flaps 20, 30 transition into a relatively flat surface along their respective widths and coil towards the needle 104 to cover the needle tip 110. The portions of the flaps 20, 30 that do not undergo a change in state can remain in the extended configuration. For example, because of the increased rigidity from the sidewalls 40, because of no rolling to create bi-stable phases or both, portions of the flaps that border the sidewalls may not experience a phase change. That is, the sidewalls 40 prevent the portions of the flaps 20, 30 bordering the sidewalls from coiling.

In one embodiment, the sidewalls 40 and the gaps 45 along the flaps 20, 30 can be positioned and shaped to ensure that when portions of the flaps 20, 30 coil or undergo a phase change, the sidewalls 40 do not interfere with each other or interfere with the portions of the flaps that are intended to undergo a change. In other embodiments, the sidewalls 40 and the gaps 45 can be designed to engage each other to interlock the flaps 20, 30 together when the needle guard is in the coiled configuration to cover the needle tip 110 in the protective position.

The distal ends of the flaps 20, 30 can cooperatively cover the needle tip 110 in the protective position to prevent the needle tip 110 from re-emerging distally out the needle guard 122. If the top flap 20 is longer than the bottom flap 30, the bottom flap 30 can transition to the coiled configuration before the top flap 20 to ensure the top flap 20 covers the bottom flap 30 in the protective position. In other examples, the bottom flap is longer than the top flap and the reverse occurs.

The sequence of how the flaps transition or undergo a phase change can be controlled by selecting where to place the sidewalls 40, where the flaps contact the activator 140, the location and shape of the activator, the number of gaps, the size of the sidewalls, or combinations thereof.

The distal extension 70 at the distal end of the top flap 20 can engage with the bottom flap 30 to help secure the needle tip 110. In one example, the distal extension 70 can wrap below the bottom flap 30 to lock the needle guard 122 and secure the top flap 20 and bottom flap 30 together. In another example, an additional distal extension (not shown) can extend distally from the bottom flap 30. The additional distal extension can create additional friction between the two flaps 20, 30 to help secure the top flap 20 and bottom flap 30 together. Once the needle guard 122 covers the needle tip 110 in the protective position, the needle guard 122 can be pulled away from the activator 140 allowing the needle 104 to separate from the catheter hub 106 with the needle guard 122 covering the needle tip 110.

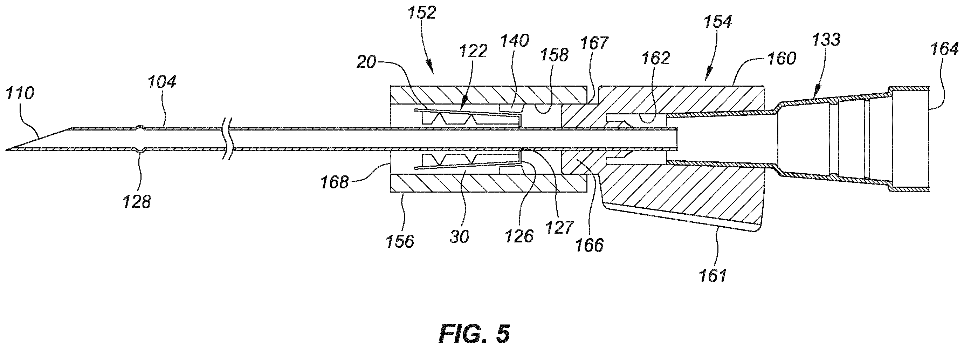

With reference now to FIG. 5, a fistula needle assembly 150 is shown for use in dialysis access and shown in a ready to use position. The present assembly comprises a first hub 152 and a second hub 154 removably engaged to one another in a ready to use position, which is a position in which the needle assembly 150 is ready for use on a patient. The second hub 154 has a needle 104 with a change in profile 128 proximal of a needle tip 110 extending therefrom. The first hub 152 comprises a body 156 defining an interior cavity 158 having a tip protector or needle guard 122 located therein. The second hub 154, which may also be referred to as a needle hub, has a body 160 defining an interior cavity 162 having a proximal opening having a vent plug 133 attached thereto. The interior cavity 162 may be referred as a blood flashback chamber and the vent plug 133 can include a vent filter 164 at a proximal end thereof for venting air or gas but not fluid, such as blood. A nose section 166 of the second hub 154 can extend into a proximal opening 168 of the first hub 152 to removably secure the two hubs 152, 154 together in the ready to use position. In other examples, the two hubs 152, 154 can removably engage one another in the ready to use position using other means, such as by gripping the external surface of the first hub 152 with a shroud on the second hub. A pair of wings 161 can extend laterally of the body 160 to provide points for securing the second hub 154 to the patient.

The tip protector 122 of the present embodiment may be similar to the tip protector discussed above with reference to FIGs. 1A-4B and has bistable configurations, phases, or states. In the present embodiment, the tip protector 122 is used with a needle 104 without an over-the-needle tube, such as a catheter tube. As shown, the tip protector 122 is located in the interior cavity 158 of the first hub 152, which has a proximal opening 166 and a distal opening 168. The interior cavity 158 defines a bore that is accessible through the proximal opening 168 and/or the distal opening 168. In other examples, the distal end of the first hub 152 can have a distal wall to partly close the distal opening 168 such that a smaller hole is provided as the distal opening that is sufficiently large for the needle 104 to pass therethrough but not the needle guard 122. The proximal opening 166 of the first hub 152 should be sufficiently large to allow the needle guard 122 to pass proximally thereof in a protective position to separate from the first hub 152, similar to the needle guard 122 of FIGs. 1A, 3A and 3B after activation and is removed from the proximal opening of the catheter hub.

Interiorly, the first hub 141 has a trigger or activator 140 for activating the needle guard 122, such as to change the state of the needle guard 122 from an extended state or first configuration to a coiled state or second configuration, as previously discussed. The trigger or activator 140 may be retained in the bore 158 by a projection 138. The activator 140 can be similar to the activator discussed elsewhere herein. In some examples, the first housing 152, and the activator140 are unitarily formed, such as by plastic injection. In other examples, the activator 140 is separately formed and subsequently assembled to the first hub 152.

In use, the needle 104 is inserted to gain access to the patient's venous system. After use, the needle 104 is retracted away from the patient by pulling on the second hub 154 in the proximal direction while the first hub 152 is held so that the needle moves relative to the first hub and the needle guard 122. The needle 104 moves until the change in profile 128 abuts a perimeter defining an opening 127 on the proximal wall 126 of the needle guard, which is smaller in dimension that the largest width at the change in profile 128 so that a physical contact is made by the change in profile at the opening 127. The needle then pulls the needle guard 122 in the proximal direction against the activator 140, which causes the needle guard to change its state from a first or extended state to a second or coiled state to cover the needle tip 110, as discussed above. The needle guard 122 then separates from the first hub 152 with the needle 104. The first hub 152 and the protected needle 104 having the needle guard 122 covering the needle tip can be disposed of via standard protocol.

Alternatively, the needle can be removed from the patient before activating the needle guard 122. This can be performed by holding the needle hub or the second hub 154 steady while advancing the first hub 152 with the needle guard 122 located therein distally relative to the needle 104 towards the needle tip 110 so that the proximal wall 126 abuts the change in profile 128. Further movement at this point forces the activator 140 inside the first hub to contact and cause the needle guard 122 to change its state from a first or extended state to a second or coiled state to cover the needle tip 110, as discussed above. Alternatively or additionally, the second hub 154 can be retracted in the proximal direction while the first hub 152 is moved in the distal direction to activate the needle guard 122.

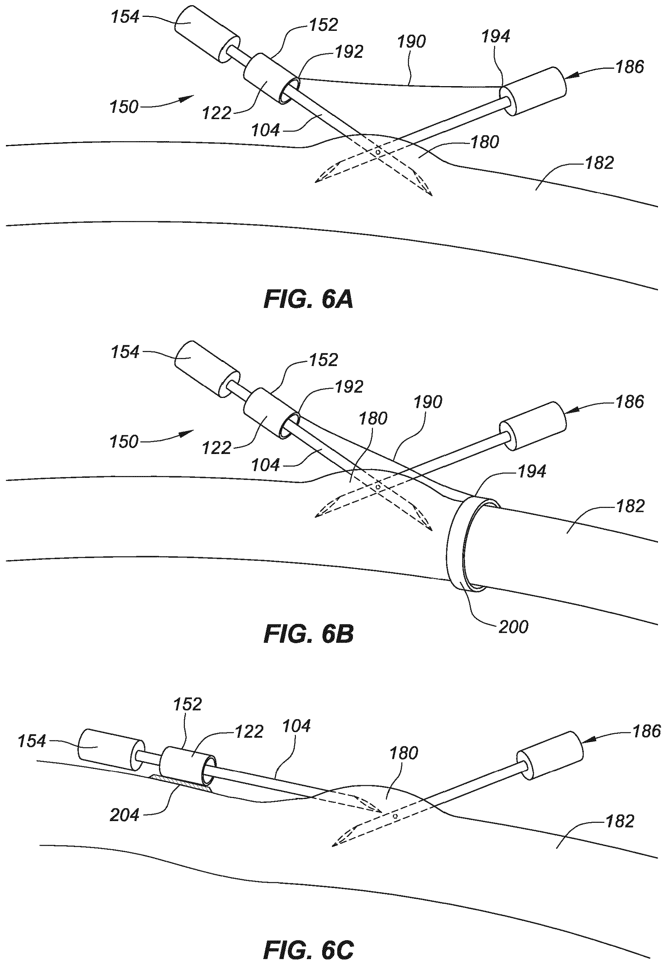

With reference now to FIG. 6A, a dialysis shunt 180 on an arm 182 with a venous needle assembly 150 and an arterial needle assembly 186 are shown. The two needle assemblies 150, 186 can be arranged in a conventional manner on an arm 182 for hemodialysis. The venous needle assembly 150 of the present embodiment can be the same as the fistula needle assembly 150 shown in FIG. 5. The arterial needle assembly 186 can be a conventional needle assembly for use in hemodialysis.

FIG. 6A shows an alternative mechanism and method for activating a needle guard 122 located inside a first hub 152 of the present disclosure. As shown, a tether 190 is connected to the first hub 152 to secure the first hub 152 while a user or practitioner retracts the needle 104 with a needle tip in the proximal direction relative to the first hub following dialysis. The user or practitioner can retract the needle 104 by gripping and moving the second hub 154 away from the first hub. This allows the change in profile 128 on the needle to be moved proximally against the proximal wall of the needle guard 122 and then pulling or moving the needle guard 122 proximally against the activator 140 to activate the needle guard, similar to the process discussed above with reference to FIG. 5 when the first hub is held steady by a user and the needle is retracted in the proximal direction relative to the first hub. The needle guard 122 of FIG. 6A therefore can change state from a first or extended state to a second or coiled state to cover the needle tip 110 by using a tether 190 to hold the first hub 152 steady and permit relative movement between the first hub 152 having the needle guard 122 located therein and the needle 104.

As shown in FIG. 6A, the tether 190 may have a first end connected to the first hub 152 and a second end 194 connected to an arterial needle assembly 186. The tether 190 may be made from any conventional prior art material, including from a fiber material, a plastic material, or a thin gauge wire. The ends 192, 194 of the tether 190 can be secured by adhesive, welding, knotting, or combinations thereof.

FIG. 6B shows a dialysis shunt 180 on an arm 182 with a venous needle assembly 150 and an arterial needle assembly 186, similar to the embodiment of FIG. 6A. In the present embodiment, one of the ends 194 of the tether 190 is connected to an anchor 200 instead of the arterial needle assembly 186. In an example, the anchor 200 can be a strap, an adhesive strip, or other securement means for holding the end 194 of the tether steady against the force of the retracting needle 104. As the needle 104 is retracted, the anchor 200 holds the tether thus preventing the first hub 152 from moving so that the needle 104 can move relative to the first hub to move the change in profile on the needle against the proximal wall of the needle guard, as discussed above. The needle guard 122 of FIG. 6B therefore can change state from a first or extended state to a second or coiled state to cover the needle tip 110 by using a tether 190 and an anchor 200 to hold the first hub 152 steady and permit relative movement between the first hub 152 having the needle guard therein and the needle 104.

FIG. 6C shows a dialysis shunt 180 on an arm 182 with a venous needle assembly 150 and an arterial needle assembly 186, similar to the embodiment of FIG. 6A. In the present embodiment, the first hub 152 is secured by an adhesive structure 204, such as a tape, a two-sided tape, an adhesive pad, or combinations thereof. As the needle 104 is retracted, the adhesive structure 204 holds the first hub 152 from moving so that the needle 104 can move relative to the first hub to move the change in profile on the needle against the proximal wall of the needle guard, as discussed above. The needle guard 122 of FIG. 6C therefore can change state from a first or extended state to a second or coiled state to cover the needle tip 110 by using an adhesive structure 204 to hold the first hub 152 steady and permit relative movement between the first hub 152 having the needle guard therein and the needle 104. In other examples, a strap or a band structure can be secured to a patient's arm and the strap or a band structure can have a receiving bay for receiving the first hub to secure the first hub and allowing relative movement between the first hub and the needle. The strap or band with the bay can wrap around the arm or can be secured to the arm with adhesive.

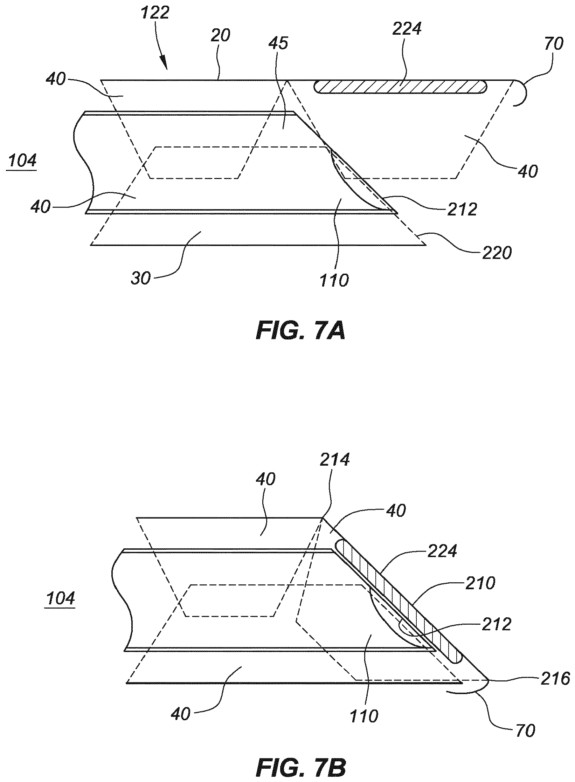

With reference now to FIG. 7A and 7B, a needle guard 122 having bistable configurations, phases, or states located inside a hub (not shown) and mounted on a needle 104 are shown in a pre-activated position and post-activated position, respectively. The needle guard 122 can be similar to the needle guards discussed elsewhere herein and can be located inside a hub having an activator, similar to the first hub 152 of FIGs. 5 and 6A-6C. The needle guard 122 can be activated to change state from a first or extended state, as shown in FIG. 7A, to a second or coiled state, as shown in FIG. 7B.

In the present embodiment, the first and second arms 20, 30 of the needle guard 122 are sized and shaped to coil into a gate 210 to close or block the bevel 212 at the needle tip 110 when the needle guard is activated by an activator. In an example, a gap 45 is created between each of two sets of sidewalls 40 on the first arm 20. Each of the gaps 45 can be axially located on the first arm so that when the first arm 20 is activated, a section of the first arm having a length is slanted to form the gate 210 for blocking the needle bevel 212. The length can be selected, such by selecting where to form the gaps 45, so that a bend 214 at or near the apex of the gap 45 extending to a tip 216 near the extension 70 is sufficiently long so as to extend across the entire bevel 212 of the needle. The sidewalls 40 on the second arm 30 can be provided with a distal mating edge 220. When the gate 210 rotates to cover the bevel, the gate can abut or contact the two distal mating edges 220 of the second arm 30.

In the present embodiment, the second arm 30 can be provided with a sidewall 40 extending from each of the two side edges of the second arm and each with a distal mating edge 220. The sidewalls 40 can be provided without any gap so as to form rigid sections to prevent the second arm from activating. In some examples, the sidewalls 40 of the second arm 30 can include one or more gaps formed at locations that will not affect the gate's ability to slant against the distal mating edges 220.

In an example, a sealing member 224 can be provided on the interior surface of the gate 210. The sealing member 224 can embody an elastic member, such as a soft rubber or silicone material. The sealing member 224 can be attached to the first arm by forming two or more tabs on the first arm that can be shaped or folded to grip the sealing member. For example, slits or cuts can be formed through the first arm that can then be folded to grab the sealing member. In other examples, adhesive or bonding can be used with or without the folded tabs to secure the sealing member 224 to the first arm. The sealing member 224 is sized and shaped and has a sufficient thickness to cover the needle bevel 212 as the gate 210 folds over the needle tip 110.

An extension 70 can be provided at a distal end of the first arm 20 for securing the gate 210 to the second arm 30 when the needle guard is activated to a protective position. Activation of the needle guard 122 can be carried out using one of several methods discussed elsewhere herein. The extension 70 can be straight, can have a hook, can include detents, can include an adhesive, or combinations thereof. The extension 70 on the first arm can be configured, such as sized and shaped, to secure against a second extension or a mating surface on the second arm.

In some examples, the needle guard 122 can include additional sealing members 224 located in the needle holding chamber of the needle guard, which is understood to include a space between the two arms and the sidewalls for accommodating the needle. The additional sealing members 224 can further assist with sealing a chamber around the needle tip and the needle bevel following activation of the needle guard over the needle tip.

In some examples, sensors can be added to the needle guard 122. For example, a strain gauge can be included to detect or sense activation of the needle guard. When the first arm, the second arm, or both bent or coil, the strain gauge can trigger a signal to notify that the needle guard has been activated. An open loop circuit can also be provided to sense when the first arm 20 and the second arm 30 contact one another, which closes the circuit when the two contact. This will in turn trigger a signal to notify that the needle guard has been activated as the first arm now contacts the second arm to close the circuit.

Methods of making and of using the needle devices and components thereof shown and described elsewhere herein are within the scope of the present disclosure.

Although limited embodiments of the needle devices and their components have been specifically described and illustrated herein, many modifications and variations will be apparent to those skilled in the art. Furthermore, it is understood and contemplated that features specifically discussed for one needle device embodiment may be adopted for inclusion with another needle device embodiment, provided the functions are compatible. Accordingly, it is to be understood that the needle devices and their components constructed according to principles of the disclosed device, system, and method may be embodied other than as specifically described herein. The disclosure is also defined in the following claims.

## Claims

1. A needle device (100), comprising:
a first hub (152) having a body defining an interior cavity (120) and having a proximal opening;
a second hub (154);
a needle (104) extending distally of the second hub (154) and through the first hub (152) in a ready to use position, said needle (104) comprising a shaft, a needle tip (110), and a change in profile (128) proximal of the needle tip (110);
a needle guard (122) located in the interior cavity (120) of the first hub (152), said needle guard (122) comprising a proximal wall (126) having a perimeter defining an opening having the needle (104) passing therethrough, a first arm (20) extending distally of the proximal wall (126) and having a width and a second arm (30) extending distally of the proximal wall (126) opposite the first arm (20) and having a width; and
wherein the first arm (20) and the second arm (30) are spaced from the needle (104) in the ready to use position and the needle guard (122) is in a first configuration, and
wherein at least the first arm (20) covers the needle tip (110) in a protective position and the needle guard (122) is in a second configuration when the needle guard (122) is activated and part of the width of the first arm (20), the second arm (30), or both the first and the second arms (20, 30) change from a first shape to a second shape having less of a curved surface.

2. The needle device (100) of claim 1, further comprising an activator (140) for activating the needle guard (122) to change from the first configuration to the second configuration.

3. The needle device (100) of claim 2, wherein the activator (140) is press fitted into the interior cavity (120) of the first hub (152).

4. The needle device (100) of claims 2 or 3, wherein the activator (140) is located inside the interior cavity (120) and prevented from moving proximally by a projection (138) extending from an interior surface of the interior cavity (120).

5. The needle device (100) of claim 4, wherein the projection (138) is an annular ring.

6. The needle device (100) of any one of claims 2 to 5, wherein the activator (140) has a tapered bore.

7. The needle device (100) of any one of claims 2 to 6, wherein the activator (140) has a rectangular bore.

8. The needle device (100) of any one of claims 1 to 7, wherein the first shape of the first arm (20) and the second arm (30) comprises an arcuate surface formed along one or more activating portions.

9. The needle device (100) of claim 8, wherein the arcuate surface is concave or convex.

10. The needle device (100) of claims 8 or 9, wherein the one or more activating portions of the first arm (20) and the second arm (30) coil towards the needle (104) in the second configuration to cover the needle tip (110).

11. The needle device (100) of any one of claims 1 to 10, wherein a distal extension (70) extends distally from the first arm (20).

12. The needle device (100) of any one of claims 1 to 11, wherein the distal extension (70) is parallel to the first arm (20).

13. The needle device (100) of any one of claims 1 to 12, wherein one or more sidewalls (40) extend from opposite sides of the first arm (20).

14. The needle device (100) of claim 13, wherein gaps are provided between the one or more sidewalls (40).

15. The needle device (100) of any one of claims 1 to 14, wherein the needle (104) is a fistula needle.

16. The needle device (100) of any one of claims 1 to 15, further comprising a tether (190) having a first end attached to the first hub (152).

17. A method of forming a needle device (100), the method comprising: providing a needle device (100) of any preceding claim.

18. A method of using a needle device (100), the method comprising:
providing a needle device (100) of any one of claims 1-16;
retracting the second hub (154);
engaging the change in profile (128) with the opening of the proximal wall (126);
triggering at least one of the first arm (20) and the second arm (30) to coil towards the needle (104) by engaging the first arm (20) and the second arm (30) with the activator (140).

19. A method according to claim 18, wherein the retracting of the second hub (154) follows successful venipuncture.
